# EUROPEAN PATENT APPLICATION

(11) **EP 4 541 796 A1**
(43) Date of publication of application: **23.04.2025**
(21) Application number: 23845377.3
(22) Date of filing: 18.07.2023
(51) Int. Cl.: C07D 491/22, A61K 31/4745, A61P 35/00, A61K 47/68

(54) **EXATECAN DERIVATIVES AND USE THEREOF**

(30) Priority: 29.07.2022 CN 202210920035
(71) Applicant: Hangzhou Adcoris Biopharma Co., Ltd, Hangzhou, Zhejiang 310056 (CN)
(72) Inventor: HUANG, Yunsheng, Hangzhou, Zhejiang 311258 (CN); LI, Yaowu, Hangzhou, Zhejiang 311258 (CN); WU, Kai, Hangzhou, Zhejiang 311258 (CN); ZHANG, Shiyi, Hangzhou, Zhejiang 311258 (CN); JIN, Xue, Hangzhou, Zhejiang 311258 (CN)
(74) Representative: Elzaburu S.L.P.
(86) International application number: PCT/CN2023/107930
(87) International publication number: WO 2024/022165

(57) **Abstract**

The present invention provides a series of structurally novel exatecan derivatives, pharmaceutically acceptable salts, stereoisomers or prodrugs thereof, and use thereof in the field of anti-tumor. The exatecan derivatives have a structure represented by formula (I).

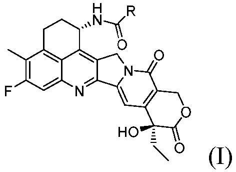

## Description

### TECHNICAL FIELD

The present disclosure relates to the field of biomedicine, specifically to exatecan derivatives and use thereof.

### BACKGROUND

Exatecan is a fully synthetic derivative of the natural product camptothecin, and has a very strong inhibitory activity against topoisomerase Top1, promoting apoptosis of tumour cells, and possessing broad-spectrum anti-tumour activities. Because of its high toxic and side effects, the application of exatecan in the field of antitumour drugs has been limited. The antibody-drug conjugate DS-8201a (ADC), formed with exatecan hydroxyethylamide derivative (DXd) (as the toxin) and trastuzumab (Herceptin), has shown very good therapeutic effects on tumors expressing Her2. DS-8201a is a high-performance ADC anti-cancer drug. In addition to Herceptin, ADC drugs targeting other antigens such as B7-H3 and Trop2 are also being extensively studied.

Based on the maintenance of the pharmacodynamic skeleton of exatecan, the present disclosure derives exatecan with hydroxyamine and hydrazine to create a series of exatecan derivatives with coupling sites which have high anti-tumor activity, low toxicity, and solubility in water, and possess potential application value as anti-tumor monotherapy or antibody-drug conjugates.

### SUMMARY OF THE INVENTION

The present disclosure provides a series of structurally novel exatecan derivatives, pharmaceutically acceptable salts, stereoisomers or prodrugs, and use thereof in the field of anti-tumor.

In one aspect, the present disclosure provides a compound represented by formula (I) or a pharmaceutically acceptable salt, a stereoisomer, or a prodrug thereof: 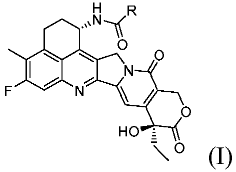 wherein,
R is selected from -Z-R₁, wherein, Z is a single bond or C=O;
R₁ is selected from -(CH₂)ₙNRₐR_{b}, -CH₂ORₐ, -NORₐ, -(CH₂)ₙONRₐR_{b} or -R₃; n is selected from 0, 1, 2 or 3;
Rₐ, R_{b} are each independently selected from hydrogen, hydroxy, amino, C₁-C₆ alkyl, C₁-C₆ alkylamino, aminoC₁-C₆ alkyl or C₁-C₆ alkoxy;
R₃ is selected from 3 to 6 membered cycloalkyl or 3 to 6 membered heterocycloalkyl containing N, O, or S heteroatoms, R₃ is optionally further substituted with R_{c};
R_{c} is selected from hydrogen, hydroxy, amino, C₁-C₆ alkyl, C₁-C₆ alkylamino or C₁-C₆ alkoxy; provided that -Z-R₁ is not -NH₂, -CH₂OH, -CH₂NH₂ or -CH₂OCH₂NH₂.

In one embodiment, R₁ is not -CH₂ONH₂ or hydroxyoxetanyl.

In one embodiment, R₁ is selected from -NRₐR_{b}, -CH₂NRₐR_{b}, -CH₂ORₐ, -NORₐ, -(CH₂)ₙONRₐR_{b} or -R₃.

Preferably, R₁ is selected from -NRₐR_{b}, -C(O)NRₐR_{b}, -CH₂NRₐR_{b}, -CH₂ONRₐR_{b} or -R₃.

In one embodiment, Rₐ, R_{b} are each independently selected from hydrogen, hydroxy, amino, C₁-C₆ alkyl, C₁-C₆ alkylamino or C₁-C₆ alkoxy.

Preferably, Rₐ, R_{b} are each independently selected from hydrogen, hydroxy, amino, methyl, methylamino or methoxy.

In one embodiment, R₃ is selected from 3 to 6 membered heterocycloalkyl containing N, O, or S heteroatoms, R₃ is optionally further substituted with R_{c}; the R_{c} is selected from hydrogen, hydroxy, amino, C₁-C₆ alkyl, C₁-C₆ alkylamino or C₁-C₆ alkoxy.

In one embodiment, R₁ is selected from -NHNH₂, -N(CH₃)NH₂, -N(CH₃)NHCH₃, -C(O)NHNH₂, -C(O)N(CH₃)NH₂, -CH₂NHOH, -CH₂NHOCH₃, -CH₂ONH₂, -CH₂ONHCH₃, -CH₂NCH₃NH₂, - CH₂N(CH₃)NHCH₃, -CH₂N(CH₃)OH, -CH₂NHOCH₂CH₃, hydroxyoxetanyl ( 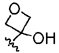 ), aminooxetanyl ( 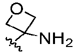 ).

In one embodiment, R₁ is selected from -NHNH₂, -N(CH₃)NH₂, -N(CH₃)NHCH₃, -C(O)NHNH₂, -C(O)N(CH₃)NH₂, -CH₂NHOH, -CH₂NHOCH₃, -CH₂ONHCH₃, -CH₂NCH₃NH₂, - CH₂N(CH₃)NHCH₃, -CH₂N(CH₃)OH, -CH₂NHOCH₂CH₃, aminooxetanyl ( 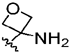 ).

In one embodiment, the compound represented by formula (I) is the compound represented by formula (Ia): 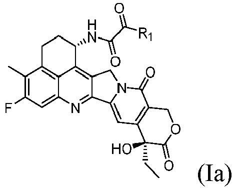 wherein, R₁ is defined the same as the compound represented by formula (I).

Preferably, R₁ is -NHNH₂ or -N(CH₃)NH₂.

In one embodiment, the compound represented by formula (I) is the compound represented by formula (Ib): 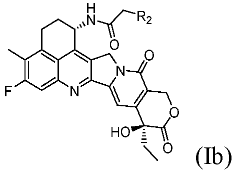 wherein, R₂ is selected from -NHOH, -ONH₂, -NHO(C₁-C₃ alkyl), -ONH(C₁-C₃ alkyl), -N(C₁-C₃ alkylamino), -N(C₁-C₃ alkyl)NH(C₁-C₃ alkyl), -N(C₁-C₃ alkyl)OH or -NHO(C₁-C₃ alkyl). Preferably, R₂ is selected from -NHOH, -NHOCH₃, ONH₂, -ONHCH₃, -NCH₃NH₂, - N(CH₃)NHCH₃, -N(CH₃)OH or -NHOCH₂CH₃.

In one embodiment, R₂ is selected from -NHOH, -NHO(C₁-C₃ alkyl), -ONH(C₁-C₃ alkyl), -N(C₁-C₃ alkylamino), -N(C₁-C₃ alkyl)NH(C₁-C₃ alkyl), -N(C₁-C₃ alkyl)OH or -NHO(C₁-C₃ alkyl). Preferably, R₂ is selected from -NHOH, -NHOCH₃, -ONHCH₃, -NCH₃NH₂, -N(CH₃)NHCH₃, - N(CH₃)OH or -NHOCH₂CH₃.

In one embodiment, the compound represented by formula (I) is the compound represented by formula (Ic): 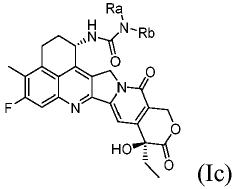 wherein, Rₐ, R_{b} are defined the same as the compound represented by formula (I); provided that Rₐ, R_{b} are not both H.

Preferably, Rₐ, R_{b} are each independently selected from hydrogen, amino, C₁-C₃ alkyl, C₁-C₃ alkylamino.

Preferably, -NRₐR_{b} is selected from -NHNH₂, -N(CH₃)NH₂ or -N(CH₃)NHCH₃.

In one embodiment, the compound represented by formula (I) is the compound represented by formula (Id): 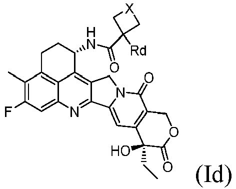 wherein, X is CH₂, NH, O or S.

Preferably, X is CH₂ or O; more preferably, X is O.

In one embodiment, R_{c} is selected from hydrogen, hydroxy, amino, C₁-C₆ alkyl, C₁-C₆ alkylamino or C₁-C₆ alkoxy.

Preferably, R_{c} is selected from hydroxy or amino, more preferably amino.

In one embodiment, the compound represented by formula (I) is selected from the following compounds:

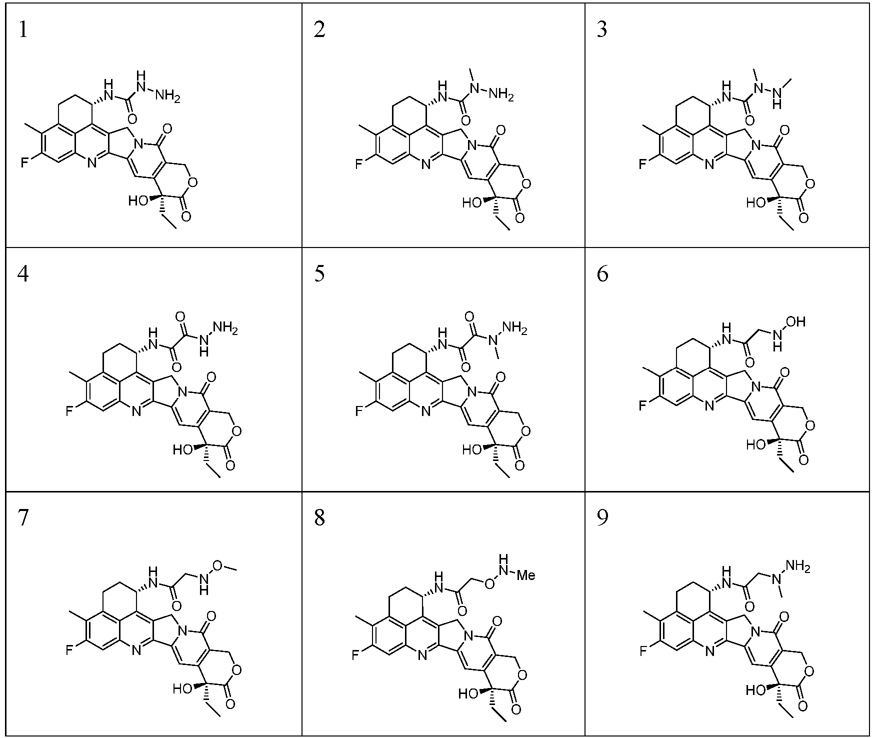

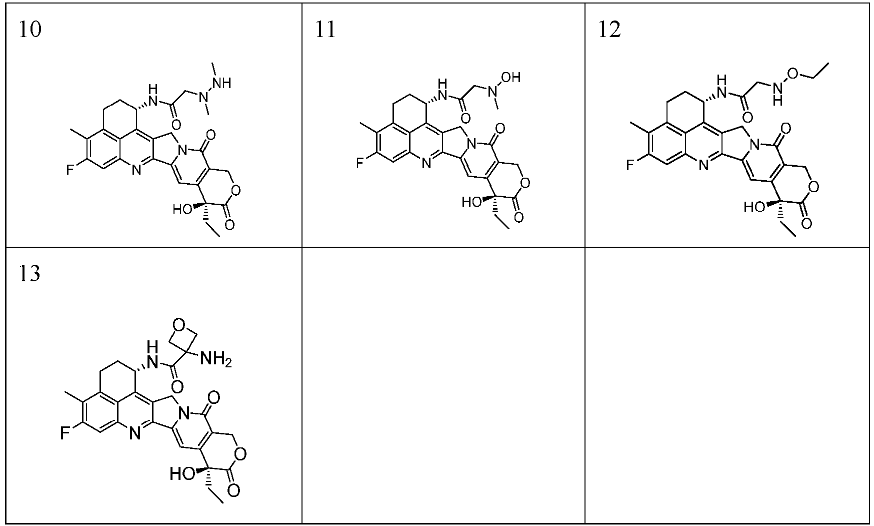

Notably, when R₁ is -CH₂ONH₂, the IC₅₀ values for growth inhibition of OE33 cells with compound 8 (R₁ is -CH₂ONHCH₃) are below 10 nM. However, the IC₅₀ value of compound 8 is still significantly lower than the IC₅₀ value of the compounds of exatecan derivatives afforded when R₁ is -CH₂ONH₂.

In another aspect, the present disclosure provides a method for preparing any one of the above compounds, selected from the following reaction schemes:

### Reaction scheme 1:

Exatecan reacts with N,N'-carbonyldiimidazole, and then reacts with hydrazine or substituted hydrazine to afford a corresponding N-aminourea exatecan derivative.

For example,

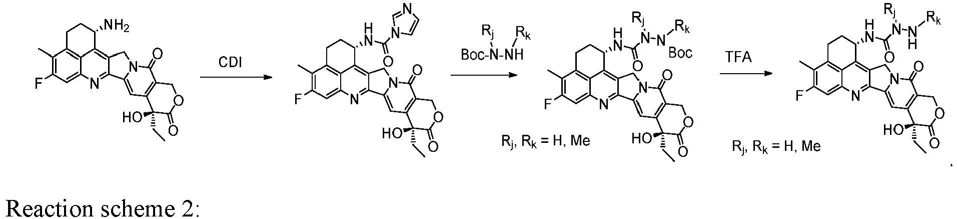

Substituted hydrazine reacts with oxalyl chloride, and then reacts with exatecan to afford a corresponding oxamide hydrazine exatecan derivative.

For example,

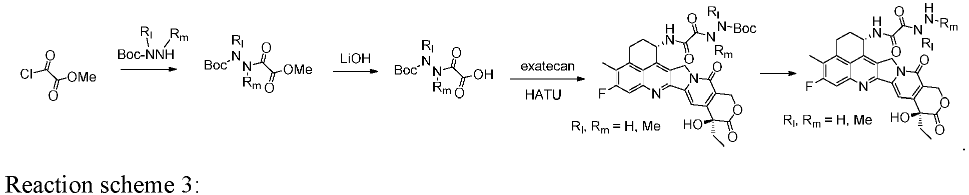

Exatecan reacts with bromoacetic acid to obtain bromoacetyl exatecan, and bromoacetyl exatecan condenses with an amine compound to afford a corresponding amide exatecan derivative.

For example,

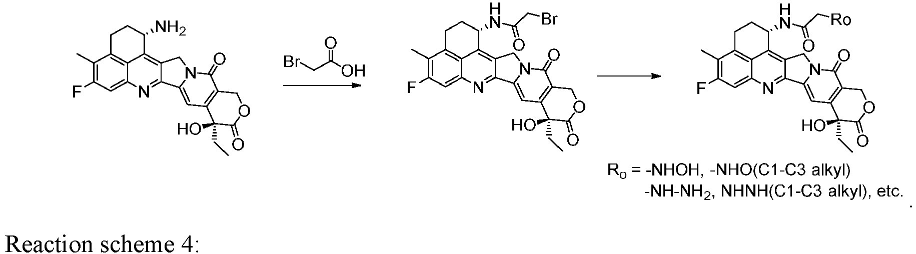

Exatecan reacts directly with a carboxylic acid compound to afford a corresponding amide exatecan derivative.

For example, 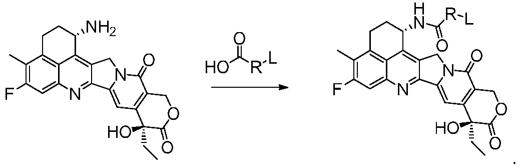 wherein, R, L are defined the same as the compound represented by formula (I).

In a further aspect, the present disclosure provides a drug-linker compound represented by formula (II) or a pharmaceutically acceptable salt, a stereoisomer, or a prodrug thereof: 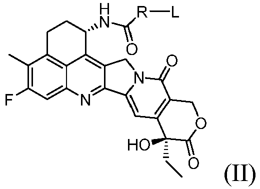 wherein, R is defined the same as the compound represented by formula (I).

In one embodiment, L is -L₁-Q-L₂; R is connected to L₁ moiety.

Wherein, L₁ is selected from 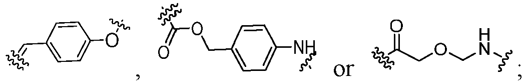 wherein, the position shown as " 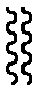 " indicates attachment to the R group, and the position shown as " 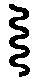 " indicates attachment to the Q group.

In one embodiment, L₂ is selected from 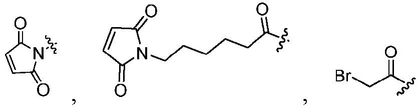 or

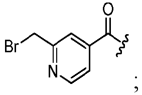

In one embodiment, Q is selected from Val-Cit, Val-Ala, Ala-Ala-Asn, Gly-Gly-Phe-Gly, Gly-Lys, Gly-Gly-lys, (CH₂)ₘ₁O(CH₂)ₘ₂, (CH₂)ₘ₃, wherein, m1 and m2 are each independently selected from an integer of 1-4.

Preferably, m1 is 2.

Preferably, m2 is 2.

Preferably, m3 is 6.

In one embodiment, Q is selected from Val-Cit, Gly-Gly-Phe-Gly, (CH₂)₂O(CH₂)₂ or (CH₂)₆; preferably, L₁ is

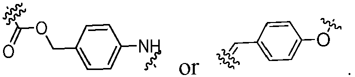

Preferably, L₂ is

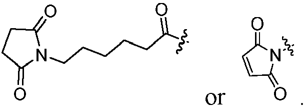

In a further aspect, the present disclosure provides an antibody-drug conjugate compound represented by formula (III) or a pharmaceutically acceptable salt thereof: 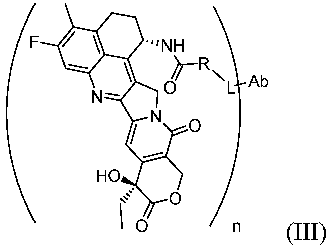 wherein, R is defined the same as formula (I), L is defined the same as formula (II).

Ab is an antibody for a tumor associated antigen, n is selected from an integer of 1-8.

Preferably, the tumor associated antigen is selected from Her2, Trop2, 5T4, ROR1 or B7-H3.

Preferably, Ab is Pertuzumab.

In one embodiment, the antibody-drug conjugate compound represented by formula (III) is selected from the following compounds:

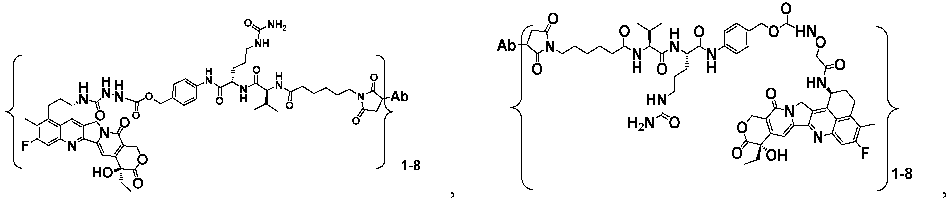

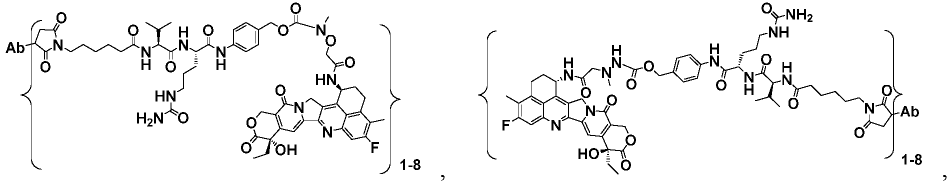

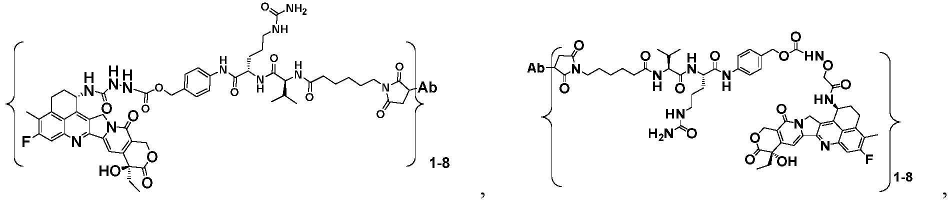

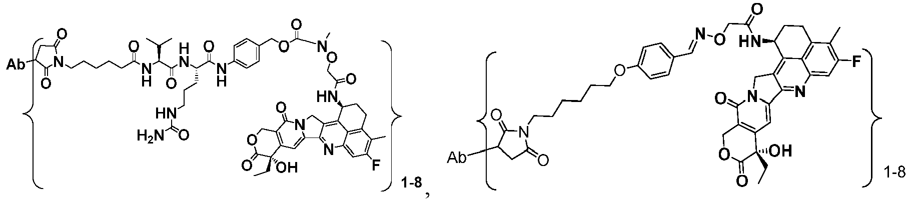

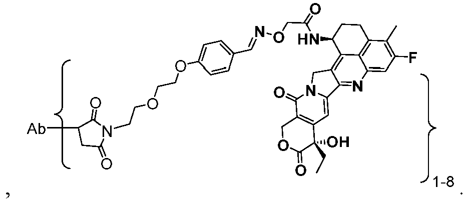

Preferably, the antibody-drug conjugate compound represented by formula (III) is selected from the following compounds:

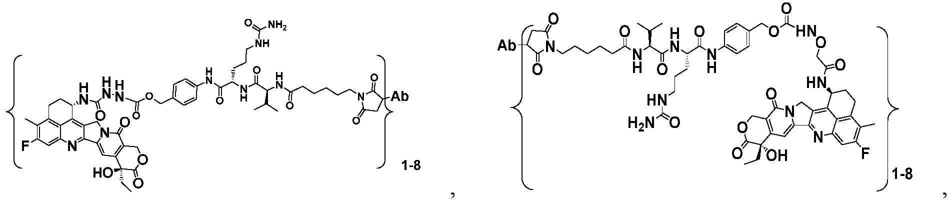

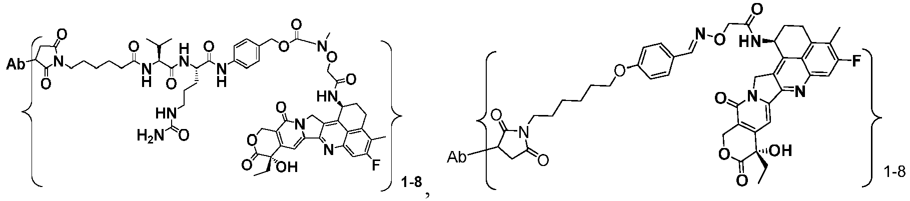

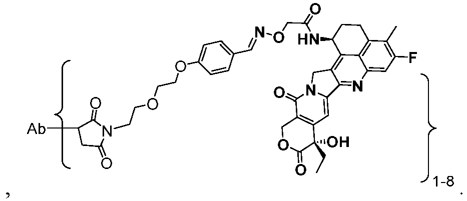

In a further aspect, the present disclosure provides a pharmaceutical composition comprising the compound, the drug-linker compound or pharmaceutically acceptable salt, stereoisomer, prodrug thereof or the antibody-drug conjugate mentioned above.

The pharmaceutical composition further comprises a pharmaceutically acceptable excipient.

In a further aspect, the present disclosure provides a use of the compound, the drug-linker compound or pharmaceutically acceptable salt, stereoisomer, prodrug thereof or the antibody-drug conjugate, the pharmaceutical composition mentioned above in the manufacture of a medicament for treating a cancer.

Preferably, the cancer includes gastric cancer, esophageal cancer, breast cancer and lung adenocarcinoma.

In another aspect, the present disclosure provides a method for treating a cancer comprising the step of administering to a patient in need thereof the compound or pharmaceutically acceptable salt, stereoisomer, prodrug thereof or the antibody-drug conjugate or the pharmaceutical composition mentioned above.

In one embodiment, the compound or pharmaceutically acceptable salt, stereoisomer, prodrug thereof, antibody-drug conjugate or pharmaceutical composition mentioned above is administered in a therapeutically effective amount.

### DETAILED DESCRIPTION OF THE EMBODIMENTS

### I. defination

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be open-ended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclusure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

The compound of the present disclosure also includes tautomeric forms. Tautomeric forms arise from the exchange of one single bond with an adjacent double bond and together with the transfer of one proton.

As used herein, "pharmaceutically acceptable salt" refers to the salt of the corresponding amine compound with an inorganic or organic acid, or the salt of the corresponding carboxyllic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid and so on; organic acids include but are not limited to acetic acid, propionic acid, butyric acid, benzoic acid, methanesulfonic acid, p-toluenesulfonic acid, oxalic acid, succinic acid, lactic acid, citric acid, succinic acid, gluconic acid, maleic acid, fumaric acid, tartaric acid and so on; alkali metal or alkaline earth metal salts include but are not limited to sodium, potassium, calcium, magnesium salts and so on; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids and so on.

As used herein, "prodrug" refers to the form in which a compound is metabolized or simply chemically altered in the body of a patient after the compound has been introduced into the human body in a suitable mode of administration to the compound contained in general formula 1 of the present invention, as well as the corresponding salt. prodrugs of the compounds include, but are not limited to, various carboxylate esters, carbonate esters, phosphate esters, sulfate esters, sulfonate esters, amino acid esters, gluconate esters, as well as various amides, acetals, hemiacetals, amidocarbonates and so on.

A numerical range as used herein refers to each integer within the given range. For example,"C1-C6" refers to the group that can have 1 carbon atom, 2 carbon atoms, 3 carbon atoms, 4 carbon atoms, 5 carbon atoms or 6 carbon atoms; "C3-C6" refers to the group that can have 3 carbon atoms, 4 carbon atoms, 5 carbon atoms or 6 carbon atoms.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "alkyl" refers to saturated aliphatic hydrocarbon groups, which is a straight or branched chain group containing 1 to 20 carbon atoms, preferably the alkyl containing 1 to 8 carbon atoms, more preferably 1 to 6 carbon atoms, and most preferably 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 2,2-diethylhexyl, 2,2-diethylhexyl, and various branched isomers thereof, etc., more preferably, a lower alkyl containing 1 to 6 carbon atoms, non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl can be substituted or unsubstituted, and when substituted, substituents can be present at any available point of attachment, the substituents preferably being one or more of the following groups, independently selected from alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl, or carboxylate group, with methyl, ethyl, isopropyl, tert-butyl, halogenated alkyl, deuterated alkyl, alkoxy-substituted alkyl, and hydroxy-substituted alkyl being preferred for the present disclosure. The term "heterocyclyl" refers to a saturated or partially unsaturated monocyclic or multicyclic cyclic hydrocarbon substituent containing 3 to 20 ring atoms, wherein one or more ring atoms are heteroatoms selected from nitrogen, oxygen, or S(O)m (wherein m is an integer from 0 to 2), but excluding ring moieties of-O-O-, -O-S-, or-S-S-, the remaining ring atoms being carbon, preferably containing 3 to 12 ring atoms, of which 1 to 4 are heteroatoms; more preferably containing 3 to 8 ring atoms; most preferably containing 3 to 8 ring atoms; further preferably containing 3 to 8 membered heterocyclic with 1 to 3 nitrogen atoms, optionally substituted with 1 to 2 oxygen atoms, sulfur atoms, oxo, including nitrogen-containing monocyclic heterocyclic group, nitrogen-containing spiroheterocyclic group or nitrogen-containing fused heterocyclic group.

The term "aryl" refers to a 6 to 14 membered all-carbon monocyclic or fused polycyclic (rings sharing adjacent pairs of carbon atoms) group having a conjugated π electron system, preferably 6 to 12 membered, such as phenyl and naphthyl.

Aryl groups can be substituted or unsubstituted. When substituted, the substituents are preferably one or more of the following groups, independently selected from: alkyl, alkenyl, alkynyl, alkoxy, alkylthio, alkylamino, halogen, thio, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkoxy" refers to -O-(alkyl) and -O-(unsubstituted cycloalkyl), wherein the definition of alkyl is as described above. Non-limiting examples of alkoxy include: methoxy, ethoxy, propoxy, butoxy, cyclopropoxy, cyclobutoxy, cyclopentoxy, cyclohexoxy. Alkoxy can be optionally substituted or unsubstituted, and when substituted, the substituents are preferably one or more of the following groups, independently selected from: alkyl, alkenyl, alkynyl, alkoxy, alkylthio, alkylamino, halogen, thio, hydroxy, nitro, chloro, cycloalkyl, heterocycloalkyl, aryl, heteroaryl, cycloalkoxy, heterocycloalkoxy, cycloalkylthio, heterocycloalkylthio, carboxyl, or carboxylate group.

The hydrogen atoms according to the present disclosure can be substituted by the isotope deuterium thereof.

The term "substituted" refers to one or more hydrogen atoms in a group, preferably up to 5, and more preferably 1-3 hydrogen atoms, being independently substituted with the corresponding number of substituents. It goes without saying that the substituents are only in their possible chemical positions, and those skilled in the art can determine (through experiment or theory) which substitutions are possible or impossible without undue effort. For example, combinations such as an amino or hydroxy having a free hydrogen with a carbon atom having an unsaturated (e.g., olefinic) bond may be unstable.
" 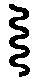 " refers to the chemical bond junction.

Medicament or pharmaceutical composition

As used herein, "pharmaceutically acceptable salt" refers to the salt of the corresponding amine compound with an inorganic or organic acid, or the salt of the corresponding carboxyllic acid compound with an alkali metal or alkaline earth metal, or with an organic amine. Wherein, inorganic acids include but are not limited to hydrochloric acid, hydrobromic acid, hydroiodic acid, sulfuric acid, phosphoric acid and so on; organic acids include but are not limited to acetic acid, propionic acid, butyric acid, benzoic acid, methanesulfonic acid, p-toluenesulfonic acid, oxalic acid, succinic acid, lactic acid, citric acid, succinic acid, gluconic acid, maleic acid, fumaric acid, tartaric acid and so on; alkali metal or alkaline earth metal salts include but are not limited to sodium, potassium, calcium, magnesium salts and so on; organic amine salts include, but are not limited to, salts composed of ammonia, methylamine, ethylamine, propylamine, isopropylamine, dimethylamine, diethylamine, trimethylamine, triethylamine, tert-butylamine, ethylenediamine, ethanolamine, diethanolamine, triethanolamine, morpholine, piperidine, piperazine, amino acids and so on.

As used herein, "prodrug" refers to the form in which a compound is metabolized or simply chemically altered in the body of a patient after the compound has been introduced into the human body in a suitable mode of administration to the compound contained in the general formula 1 of the present invention, as well as the corresponding salt. prodrugs of the compounds include, but are not limited to, various carboxylate esters, carbonate esters, phosphate esters, sulfate esters, sulfonate esters, amino acid esters, gluconate esters, as well as various amides, acetals, hemiacetals, amidocarbonates and so on.

The drugs or pharmaceutical compositions of the present disclosure can be administered orally, topically, parenterally, or mucosally (e.g., buccally, by inhalation, or rectally) in dosage unit formulations comprising conventional non-toxic pharmaceutically acceptable carriers. It is often desirable to use orally. The active agent can be administered orally in the form of capsules, tablets, etc. (see Remington: The Science and Practice of Pharmacy, 20th Edition).

For oral administration in the form of tablets or capsules, the active pharmaceutical ingredients can be combined with non-toxic, pharmaceutically acceptable excipients such as binders (for example, pregelatinized corn starch, polyvinyl pyrrolidone, or hydroxypropyl methylcellulose); fillers (for example, lactose, sucrose, glucose, mannitol, sorbitol, and other reducing and nonreducing sugars, microcrystalline cellulose, calcium sulfate, or calcium hydrogen phosphate); lubricants (for example, magnesium stearate, talc, or silica, stearic acid, sodium stearyl fumarate, glyceryl behenate, calcium stearate, etc.); disintegrants (for example, potato starch or sodium starch glycolate); or wetting agents (for example, sodium lauryl sulfate), colorants and flavorings, gelatin, sweeteners, natural and synthetic gums (such as acacia gum, tragacanth gum, or alginates), buffering salts, carboxylmethyl cellulose, polyethylene glycol, waxes, etc. For oral administration in liquid form, the pharmaceutical ingredients can be combined with non-toxic, pharmaceutically acceptable inert carriers (for example, ethanol, glycerin, water), anti-sedimentation agents (for example, sorbitol syrup, cellulose derivatives, or hydrogenated edible fats), emulsifiers (for example, lecithin or acacia gum), non-aqueous carriers (for example, almond oil, oil esters, ethanol, or fractionated vegetable oils), preservatives (for example, methyl p-hydroxybenzoate or propyl p-hydroxybenzoate or sorbic acid), etc. Stabilizers such as antioxidants (BHA, BHT, propyl gallate, sodium ascorbate, citric acid) can also be added to stabilize the dosage form.

Tablets containing the active compound can be coated using methods well-known in the field. The compositions of the present disclosure, which include the compound represented by formula I as the active compound, can also be introduced into small beads, microspheres, or microcapsules, for example, constructed with polyglycolic acid/lactic acid (PGLA). Liquid formulations for oral administration can take the forms of e.g. solution, syrup, emulsion, or suspension, or they can be presented as dry products that are reconstituted with water or other suitable excipients before use. Formulations for oral administration can be suitably formulated to provide controlled or delayed release of the active compound.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, and suspending agents.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" refers to a biopharmaceutical product that composes a small molecule drug linked to a monoclonal antibody through a chemical linker, in which the monoclonal antibody is used as a carrier to transport the small molecule drug to its target cells. As used herein, the terms "reduce," "inhibit," "alleviate," or "decrease" are relative to controls. Those skilled in the art will easily determine the appropriate control for each experiment. For example, a diminished response in subjects or cells treated with the compound is compared to the response in subjects or cells not treated with the compound.

Unless otherwise specified, the raw materials and equipment used in the specific embodiments of the present disclosure are known products that can be obtained by purchasing commercial products.

### Abbreviations

CDI: N,N' -carbonyldiimidazole; EA: ethyl acetate; DCM: dichloromethane; HATU: 2-(7-azabenzotriazolyl)-N,N,N',N'-tetramethyluronium hexafluorophosphate; DIPEA: N,N-diisopropylethylamine; NMP: N-methyl pyrrolidone; HOBt: 1-hydroxybenzotriazole;
Val: valine, the structural formula thereof is 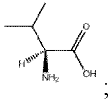 Cit: citrulline, the structural formula thereof is 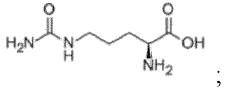 Gly: glycine, the structural formula thereof is 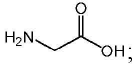 Phe: phenylalanine, the structural formula thereof is 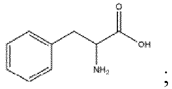 Fmoc: benzyl threonine phosphate, the structural formula thereof is 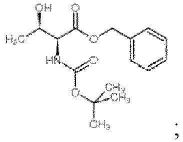 Val-Cit: 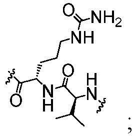 Gly-Gly-Phe-Gly: 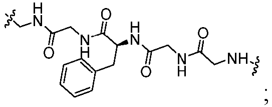
Val-Ala, Ala-Ala-Asn, Gly-Lys, Gly-Gly-lysnd and other linkage chains can be connected by amino acid condensation methods well-known in the art.

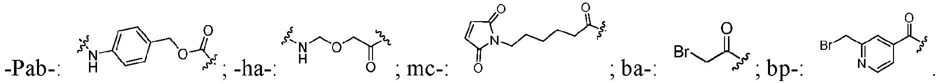

### II.Example

ADCs prepared in the present examples used, but not limited to, patuzumab (PERTUZUMAB), which has the following heavy chain amino acid sequence (SEQ ID NO: 1):

EVQLVESGGGLVQPGGSLRLSCAASGFTFTDYTMDWVRQAPGKGLEWVADVNPNSGGS
IYNQRFKGRFTLSVDRSKNTLYLQMNSLRAEDTAVYYCARNLGPSFYFDYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKLTV
DKSRWQQGNVFSCSVMHEALHNHYTQKSLSLSPG

The light chain amino acid sequence is as follows (SEQ ID NO: 2):

DIQMTQSPSSLSASVGDRVTITCKASQDVSIGVAWYQQKPGKAPKLLIYSASYRYTGVPS
RFSGSGSGTDFTLTISSLQPEDFATYYCQQYYIYPYTFGQGTKVEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC

### Example 1: Exatecan-N-aminourea (1)

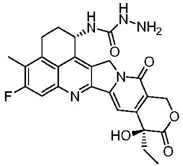

In 1mL of DMF, exatecan mesylate (30 mg, 56.44 µmol) and triethylamine (11.42 mg, 112.88 µmol) were added, and the mixture was stirred until homogeneous. Then, CDI (9.15 mg, 56.44 µmol) was added, and the mixture was reacted under argon protection for 1 h at room temperature, then triethylamine (80 mg) and hydrazine hydrochloride (19.33 mg, 0.28 mmol) were added sequentially, and the mixture was stirred to react for 10 h at room temperature. 50 mL of EA was added, and the mixture was washed with saturated brine, extracted, phaseseparated, dried over anhydrous sodium sulfate, then separated and purified by silica gel column chromatography to afford Compound 1 (25mg, yield 90%); ¹H NMR (500 MHz, DMSO-d₆) δ 7.77 (d, J=10.9 Hz, 1H), 7.54 (s, 1H), 7.30 (s, 1H), 6.52 (m, 1H), 5.41 (m, 2H), 5.39-5.33 (m, 1H), 5.22 (m, J=7.0 Hz, 2H), 3.15 (m, J=13.6, 6.9 Hz, 2H), 3.06 (s, 2H), 2.38 (s, 3H), 2.24-2.11 (m, 2H), 1.86 (m, J=21.4, 7.0 Hz, 2H), 0.87 (t, J=7.3 Hz, 3H); LCMS: (M+1)⁺ 493.98 (calculated value: 493.18).

### Example 2: Exatecan-N-methyl-N-aminourea (2)

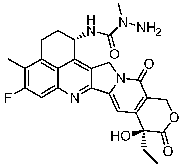

In 1mL of DMF, exatecan mesylate (30 mg, 56.44 µmol) and triethylamine (11.42 mg, 112.88 µmol) were added, and the mixture was stirred until homogeneous. Then CDI (9.15 mg, 56.44 µmol) was added, and the mixture was reacted under argon protection for 1 h at room temperature, then triethylamine (80 mg) and 1-Boc-2-methylhydrazine (45.05 mg, 0.3 mmol) were added sequentially, and the mixture was stirred to react for 10 h at room temperature. 50 mL of EA was added, and the mixture was washed with saturated brine, extracted, phaseseparated, dried over anhydrous sodium sulfate, then separated and purified by silica gel column chromatography to afford the Boc-product. Then the Boc-product was dissolved in 1mL of DCM, then a 0.5 mL solution of 4 M HCl in ethyl acetate was added, and the mixture was stirred to react for 30 min at room temperature to afford Compound 2 (22 mg, yield 80%); ¹H NMR (500 MHz, DMSO-d₆) δ 7.81 (d, *J*=10.9 Hz, 1H), 7.32 (s, 1H), 6.54 (s, 1H), 5.43 (m, *J*=18 Hz, 3H), 5.29 (d, *J*=4.1 Hz, 2H),3.29 (s, 3H) 3.17 (t, *J*=6.4 Hz, 2H), 2.41 (d, *J*=1.7 Hz, 3H), 2.20 (m, *J*=13.0, 6.9 Hz, 2H), 1.86 (m, *J*=20.7, 13.7, 6.8 Hz, 2H), 0.87 (t, *J*=7.3 Hz, 3H); LCMS: (M+1)⁺ 508.02 (calculated value: 507.19).

### Example 3: Exatecan-N',N-dimethylaminourea (3)

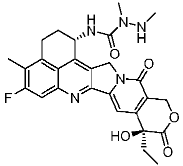

In 1mL of DMF, exatecan mesylate (30 mg, 56.44 µmol) and triethylamine (11.42 mg, 112.88 µmol) were added, and the mixture was stirred until homogeneous. Then, CDI (9.15 mg, 56.44 µmol) was added, and the mixture was reacted under argon protection for 1 h at room temperature, then TEA (80 mg) and N'N-dimethyl hydrazine hydrochloride (45.05 mg, 0.33 mmol) were added sequentially, and the mixture was stirred to react for 10 h at room temperature. 50 mL of EA was added, and the mixture was washed with saturated brine, phaseseparated, dried over anhydrous sodium sulfate, then separated and purified by silica gel column chromatography to afford Compound **3** (18 mg, yield 62%); ¹H NMR (500 MHz, DMSO-d₆) δ 7.76 (d, *J*=10.9 Hz, 1H), 7.48 (d, *J*=9.2 Hz, 1H), 7.30 (s, 1H), 6.51 (s, 1H), 5.41 (s, 2H), 5.31 (dd, *J*=17.0, 8.5 Hz, 2H), 5.16 (s, 1H), 4.64 (q, *J*=5.7 Hz, 2H), 3.09 (dt, *J*=16.8, 7.8 Hz, 2H), 3.01 (s, 3H), 2.38 (s, 3H), 2.18 (t, *J*=7.2 Hz, 3H), 1.86 (dq, *J*=19.3, 7.1 Hz, 2H), 1.47 (s, 1H), 0.86 (d, *J*=8.2 Hz, 3H); LCMS: (M+1)⁺ 522.02 (calculated value: 521.21).

### Example 4: Exatecan-N'-amino oxalyldiamine (4)

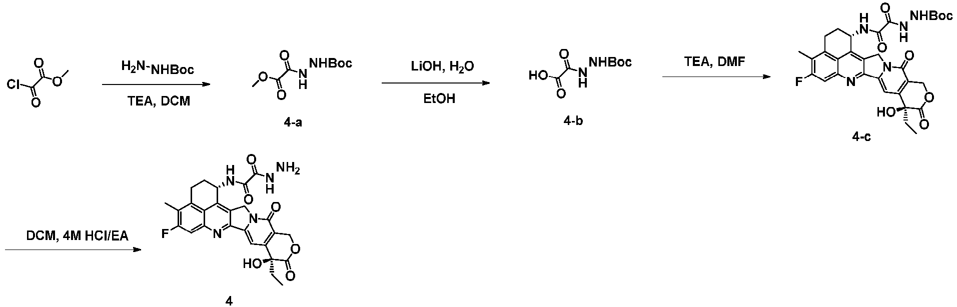

In 30mL of DCM, Boc-hydrazine (5.93 g, 44.90 mmol) and TEA (6.2 g, 61.21 mmol) were added, and the mixture was stirred until clear, then a solution of methyl oxalyl chloride (5 g, 40.81 mmol) in DCM (50 mL) was added dropwise at 0°C. The mixture was stirred to react for 1 h at room temperature, then washed with saturated brine (20mL*3), dried over anhydrous sodium sulfate, and concentrated to afford Compound 4-a, N'-Boc-N-oxalylhydrazine monomethyl ester (7.1 g, yield 79%); LCMS: (M+1)⁺ 219.01 (calculated value: 218.21).

Compound 4-a (7.1 g, 32.56 mmol) was dissolved in 70 mL of ethanol, then 32.53 mL of 1M LiOH aqueous solution was added dropwise, and the mixture was stirred overnight at room temperature. Then 0.5M HCl aqueous solution was added dropwise to adjust the pH of the reaction mixture to 5, then water and ethanol were removed under reduced pressure. 5 mL of methanol and 15 mL of DCM were added to the concentrate, and the mixture was stirred for 1 h, filtered, and the filtrate was concentrated to afford Compound 4-b, N'-Boc-N-oxalylhydrazine (6 g, yield 89%), LCMS: (M+1)⁺ 205.2 (calculated value: 204.18).

In 2 mL of DMF, exatecan mesylate (200 mg, 0.37 mmol), diisopropylethylamine (6.15 mg, 0.047 mmol), N'-Boc-N-oxalylhydrazine (164 mg, 0.8 mmol), and HATU (286 mg, 0.75 mmol) were added sequentially, and the mixture was stirred to react for 4 h at room temperature. Then DMF was removed under reduced pressure, and the concentrate was added to 20 mL of DCM, and purified by silica gel column chromatography to afford Compound 4-c, exatecan-N'-Boc-amino oxalyldiamine (180 mg, yield 77%); LCMS: (M+1)⁺ 622.1 (calculated value: 621.22).

Exatecan-N'-Boc-amino oxalyldiamine (180 mg, 0.29 mmol) was dissolved in 2 mL of DCM, then a 1 mL solution of 4 M HCl in ethyl acetate was added, and the mixture was stirred to react for 30 min at room temperature and concentrated to afford Compound **4,** i.e., exatecan-N'-amino oxalyldiamine (162mg, 100%); ¹H NMR (500 MHz, DMSO-d₆) δ 9.54 (d, *J*=8.9 Hz, 1H), 7.77 (m, *J*=11.3, 4.1 Hz, 1H), 7.30 (s, 1H), 6.52 (s, 1H), 5.53 (m, *J*=8.3, 5.0 Hz, 1H), 5.39 (s, 2H), 5.12 (d, *J*=4.0 Hz, 2H), 3.23 (s, 1H), 2.38 (s, 3H), 2.28-2.15 (m, 2H), 1.85 (m, *J*=25.0, 7.2 Hz, 2H), 0.86 (t, *J*=7.4 Hz, 3H); LCMS: (M+1)⁺ 522.00 (calculated value: 521.17).

### Example 5: Exatecan N'-amino-N-methyloxalyldiamine (5)

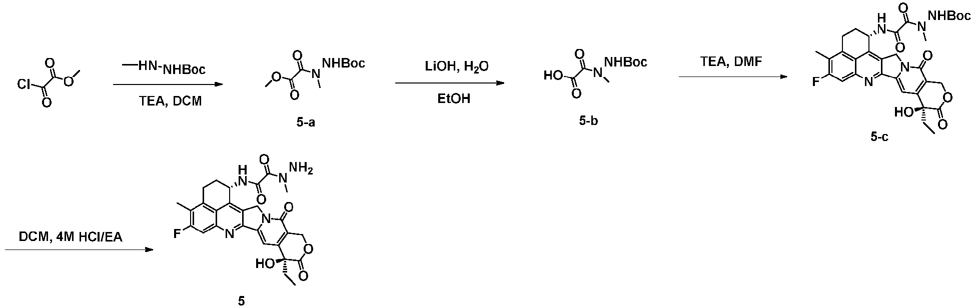

In 20 mL of DCM, 1-methyl-2-Boc hydrazine (2.4 g, 26 mmol) and TEA (1.65 g, 16.3 mmol) were added, and the mixture was stirred until clear. Then a solution of methyl oxalyl chloride (2 g, 16.3 mmol) in DCM (20 mL) was added dropwise at 0°C, and the mixture was stirred to react for 1 h at room temperature, extracted, washed with saturated brine (10mL*3), dried over anhydrous sodium sulfate, and concentrated to afford the Intermediate Compound 5-a, 1-methyl-2-Boc-oxalylhydrazine monomethyl ester (3.5 g, yield 92%), LCMS: (M+1)⁺ 233.10 (calculated value: 232.24) .

Compound 5-a (3.5 g, 15 mmol) was dissolved in 40 mL of ethanol, and 1M LiOH aqueous solution (16.37 mL) was added dropwise. The mixture was stirred overnight at room temperature, then 0.5M HCl aqueous solution was added dropwise to adjust the pH of the reaction mixture to 5, then water and ethanol were removed under reduced pressure. 2 mL of methanol and 8 mL of DCM were added to the concentrate, and the mixture was stirred for 1 h, filtered, and the filtrate was concentrated to afford Intermediate Compound 5-b, 1-methyl-2-Boc-oxalylhydrazine (3.21 g, yield 90%), LCMS: (M+1)⁺ 219.01 (calculated value: 218.21).

In 2 mL of DMF, exatecan mesylate (100 mg, 0.19 mmol), DIPEA (48.58 mg, 0.37 mmol), Compound 5-b (82 mg, 0.37 mmol), HATU (118 mg, 0.32 mmol) were added sequentially. The mixture was stirred to react for 4 h at room temperature, then DMF was removed under reduced pressure. The residue was separated and purified by silica gel column chromatography to afford Intermediate Compound 5-c, exatecan-N'-Boc-amino-N-methyloxalyldiamine (91 mg, yield 75%), LCMS: (M+1)⁺ 636.14 (calculated value: 635.65).

Compound 5-c (91 mg, 0.14 mmol) was dissolved in 2 mL of DCM, then a 1 mL solution of 4 M HCl in ethyl acetate was added, and the mixture was stirred to react for 30 min at room temperature, concentrated to afford Compound **5,** i.e., exatecan N'-amino-N-methyloxalyldiamine (85mg, yield 100%); ¹H NMR (500 MHz, DMSO-d₆) δ 8.87 (d, *J*=8.7 Hz, 1H), 7.79 (d, *J*=10.9 Hz, 1H), 7.31 (s, 1H), 5.59-5.62 (m, *J*=18 Hz,1H),5.46-5.32 (m, *J*=84Hz, 4H), 3.16-3.10 (m, 2H), 2.95 (s, 2H), 2.40 (s, 3H), 2.17 (ddq, *J*=28.2, 14.0, 5.5, 4.9 Hz, 2H), 1.86 (dp, *J*=20.9, 7.0 Hz, 2H), 0.86 (t, *J*=7.4 Hz, 3H); LCMS: (M+1)⁺ 536.03 (calculated value: 535.53).

### Example 6: 2-hydroxyaminoacetyl exatecan (6)

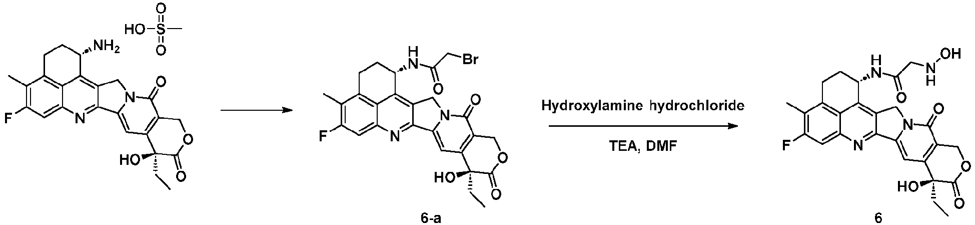

In 1 mL of DMF, exatecan mesylate (100 mg, 0.19 mmol) and TEA (25 mg, 0.24 mmol) were added, and the mixture was stirred until clear. Then, bromoacetic acid (40 mg, 0.28 mmol) and HATU (85 mg, 0.22 mmol) were added, and the mixture was stirred for 1 h at room temperature. DMF was removed under reduced pressure, and the residue was separated and purified by silica gel column chromatography to afford Intermediate **6-a,** exatecan-N-bromoacetylamine (90 mg, yield 86%), LCMS: (M+1)⁺ 557.1, (calculated value: 556.39).

In a 10 mL vial, 1 mL of DMF was added, then hydroxyamine hydrochloride (113 mg, 1.63 mmol), TEA (164 mg, 1.62 mmol), and Compound 6-a (90 mg, 0.16 mmol) were added sequentially. The mixture was stirred to react for 1 h at room temperature. DMF was removed under reduced pressure, and the residue was separated and purified by silica gel column chromatography to afford Compound **6,** i.e., 2-hydroxyaminoacetyl exatecan (90 mg, yield 86%); ¹H NMR (500 MHz, DMSO-d₆) δ 10.86-10.38 (m, 1H), 8.96 (d, *J*=8.7 Hz, 1H), 7.82 (t, *J*=11.9 Hz, 1H), 7.32 (s, 1H), 6.55 (s, 1H), 5.77-5.54 (m, 1H), 5.41 (d, *J*=10.6 Hz, 2H), 5.37-5.16 (m, 2H), 3.86 (m, *J*=11.7, 8.0 Hz, 2H), 3.31-3.02 (m, 3H), 2.40 (d, *J*=14.0 Hz, 3H), 2.29-2.07 (m, 2H), 1.86 (m, *J*=21.6, 7.2 Hz, 2H), 0.86 (t, *J*=7.3 Hz, 3H); LCMS: (M+1)⁺ 509.3 (calculated value: 508.18).

### Example 7: 2-methoxyaminoacetyl exatecan (7)

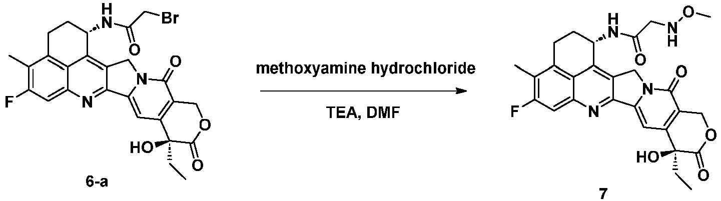

In a 10 mL vial, 1 mL of DMF was added, then methoxyamine hydrochloride (126 mg, 1.5 mmol), TEA (182 mg, 1.79 mmol), and Compound 6-a (100 mg, 0.18 mmol) were added sequentially. The mixture was stirred for 1 h at room temperature. DMF was removed under reduced pressure, and the residue was separated and purified by silica gel column chromatography to afford Compound **7,** i.e., 2-methoxyaminoacetyl exatecan (80 mg, yield 86%); ¹H NMR (500 MHz, DMSO-d₆) δ 8.50-8.46 (m, 1H), 7.80 (d, *J*=10.8 Hz, 1H), 7.30 (d, *J*=1.9 Hz, 1H), 6.83 (td, *J*=6.2, 1.8 Hz, 1H), 6.52 (d, *J*=1.9 Hz, 1H), 5.61-5.55 (m, 1H), 5.42 (s, 2H), 5.22 (s, 2H), 4.01-3.87 (m, 1H), 3.42 (d, *J*=6.1 Hz, 3H), 3.17 (t, *J*=6.4 Hz, 2H), 2.40 (s, 3H), 2.22-2.11 (m, 2H), 1.86 (dp, *J*=21.1, 7.1 Hz, 2H), 0.90-0.83 (m, 3H); LCMS: (M+1)⁺ 523.20 (calculated value: 522.19).

### Example 8: 2-methylaminooxyacetyl exatecan (8)

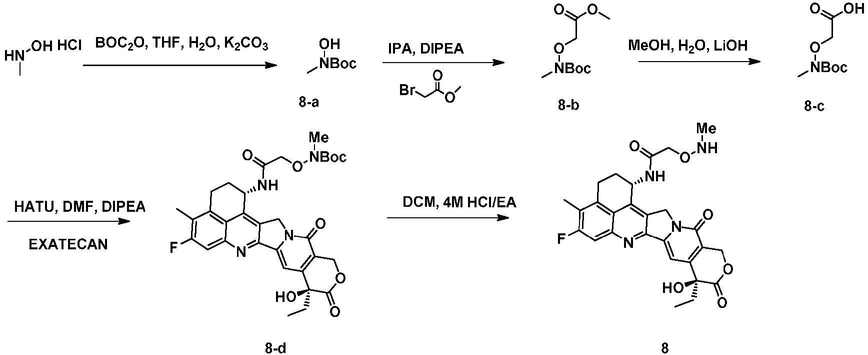

In a mixture of 40 mL of water and 40 mL of THF, N-methyl hydroxyamine hydrochloride (10 g, 119.7 mmol) was added, and the mixture was stirred until clear. Then potassium carbonate (8.27 g, 60 mmol) was added, followed by slowly dropwise adding a solution of Boc anhydride (28.75 g, 131.7 mmol) in THF (50 mL). After the dropwise addition was completed, the mixture was stirred overnight at room temperature. THF was removed under reduced pressure, and the residue was extracted with DCM (100mL*3), dried over anhydrous sodium sulfate and concentrated to afford Compound **8-a,** N-Boc-N-methylhydroxyamine (15 g, yield 88%), LCMS: (M+1)⁺ 148.01 (calculated value: 147.17).

Compound **8-a** (15 g, 101.9 mmol) was dissolved in 100 mL of isopropanol, then methyl bromoacetate (18.58 g, 121.4 mmol) and DIPEA (15.7 g, 121.7 mmol) were added. The mixture was heated to 85°C to react for 3 h under argon protection. The reaction mixture was then concentrated, and the residue was dissolved in 100 mL of EA, washed with saturated brine (30mL*3), dried over anhydrous sodium sulfate, and concentrated to afford Compound **8-b,** methyl N-Boc-N-methylaminooxyacetate (18 g, yield 81.8%), LCMS: (M+1)⁺ 220.21 (calculated value: 219.24).

Compound **8-b** (18 g, 82 mmol) was dissolved in 180 mL of methanol, then 82.2 mL of 1M lithium hydroxide aqueous solution was added, and the mixture was stirred to react at room temperature. After the reaction was completed, the reaction mixture was concentrated by removing methanol. The residue was added with 0.5 mmol/L aqueous hydrochloric acid to adjust the pH to approximately 3. The mixture was extracted with EA, and the phases were separated. The organic phase was dried, filtered, and concentrated to afford Compound **8-c,** N-Boc-N-methylamineoxyacetic acid (13.1 g, yield 77.3%), LCMS: (M+1)⁺ 206.01 (calculated value: 205.21).

In 2 mL of DMF, exatecan mesylate (100 mg, 0.19 mmol), DIPEA (48.58 mg, 0.37 mmol), Compound **8-c** (77.3 mg, 0.37 mmol), and HATU (118 mg, 0.31 mmol) were added sequentially. The mixture was stirred to react for 6 h at room temperature. DMF was removed under reduced pressure, and the residue was separated and purified by silica gel column chromatography to afford Compound **8-d,** N-Boc-N-methylaminooxyacetyl exatecan (68 mg, yield 75%), LCMS: (M+1)⁺ 623.30 (calculated value: 622.65).

Compound **8-d** (68 mg, 0.109 mmol) was dissolved in 2 mL of DCM, then a 1 mL solution of 4M hydrochloric acid in ethyl acetate was added, and the mixture was stirred to react for 30 min at room temperature, then concentrated to afford Compound **8,** i.e., 2-methylaminooxyacetyl exatecan hydrochloride (61 mg, yield 100%); ¹H NMR (500 MHz, DMSO-d₆) δ 8.93 (d, *J*=8.5 Hz, 1H), 7.79 (d, *J*=10.5 Hz, 1H), 7.30 (s, 1H), 5.60 (dt, *J*=8.3, 4.1 Hz, 1H), 5.42 (s, 2H), 5.29 (s, 2H), 4.73-4.64 (m, 2H), 3.18 (s, 2H), 2.85 (s, 3H), 2.39 (s, 3H), 2.25 (dt, *J*=8.8, 4.6 Hz, 1H), 2.19-2.10 (m, 1H), 1.85 (dp, J=21.4, 7.2 Hz, 2H), 0.86 (t, J=7.3 Hz, 3H); LCMS: (M+1)⁺ 523.10 (calculated value: 522.19).

### Example 9: 2-N-methyl-N-aminoacetyl exatecan (9)

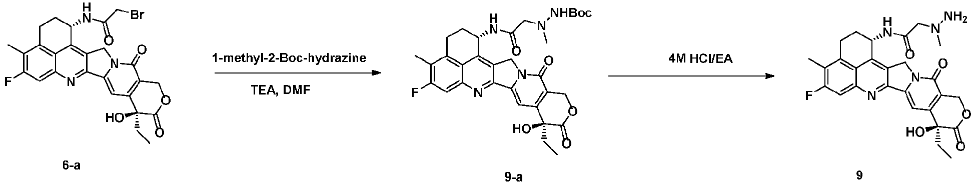

In a 10 mL vial, 1 mL of DMF was added, then 1-methyl-2-Boc hydrazine (36.41 mg, 0.25 mmol), TEA (54.6 mg, 0.54 mmol), and 6-a (100 mg, 0.18 mmol) were added sequentially. The mixture was stirred to react for 1 h at room temperature. DMF was removed under reduced pressure, and the residue was separated and purified by silica gel column chromatography to afford Compound **9-a,** 2-N-methyl-N-Bocaminoacetyl exatecan (72 mg, yield 63%), LCMS: (M+1)⁺ 622.30 (calculated value: 621.21).

Compound **9-a** (72 mg, 0.115 mmol) was dissolved in 2 mL of DCM, then a 1 mL solution of 4M hydrochloric acid in ethyl acetate was added. The mixture was stirred to react for 30 min at room temperature to afford Compound **9,** i.e., 2-N-methyl-N-aminoacetyl exatecan (64 mg, yield 100%); ¹H NMR (500 MHz, DMSO-d₆) δ 8.53 (d, *J*=8.9 Hz, 1H), 8.33 (s, 1H), 7.79 (d, *J*=10.9 Hz, 1H), 7.32-7.27 (m, 1H), 6.53 (d, *J*=1.6 Hz, 1H), 5.60 (dt, *J*=9.3, 5.1 Hz, 1H), 5.41 (s, 2H), 5.29-5.15 (m, 2H), 3.42-3.40(m, 2H), 3.38-3.37(m, 2H), 3.37 (s, 3H), 3.19 (d, *J*=5.6 Hz, 1H), 2.39 (s, 3H), 2.15 (q, *J*=6.0 Hz, 2H), 1.85 (dp, *J*=21.7, 7.0 Hz, 2H), 0.86 (t, *J*=7.3 Hz, 3H); LCMS: (M+H)⁺ 522.1 (calculated value: 521.18).

### Example 10: 2-N-methyl-N-methylaminoacetyl exatecan (10)

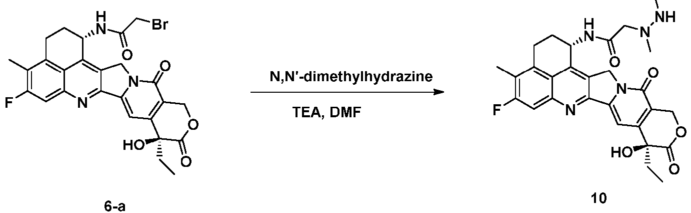

In a 10 mL vial, 1 mL of DMF was added, then 1,2-dimethyl-hydrazine dihydrochloride (71.72 mg, 0.54 mmol), TEA (91 mg, 0.89 mmol), 6-a (100 mg, 0.18 mmol) were added sequentially.

The mixture was stirred to react for 1 h at room temperature. After the reaction was completed, DMF was removed under reduced pressure, and the residue was separated and purified by silica gel column chromatography to afford Compound **10,** i.e., 2-N-methyl-N-methylaminoacetyl exatecan (85 mg, yield 88%); ¹H NMR (500 MHz, DMSO-d₆) δ 10.05 (s, 1H), 8.85 (d, *J*=9.1 Hz, 1H), 7.81 (m *J*=19.4, 10.9 Hz, 1H), 7.32 (d, *J*=11.1 Hz, 1H), 6.54 (s, 1H), 5.66-5.51 (m, 1H), 5.49-5.38(m, 2H), 5.35-5.17(m, 2H), 3.73-3.55 (m, 1H), 3.19 (m, *J*=19.8, 15.3, 9.5 Hz, 3H), 2.73 (s, 3H), 2.41 (d, *J*=9.0 Hz, 3H), 2.19 (m, *J*=31.1, 13.1, 5.4 Hz, 2H), 1.86 (m, *J*=21.7, 14.7, 7.5 Hz, 2H), 0.87 (t, *J*=7.3 Hz, 3H); LCMS: (M+1)⁺ 536.20 (calculated value: 535.22).

### Example 11: 2-N-methylhydroxyaminoacetyl exatecan (11)

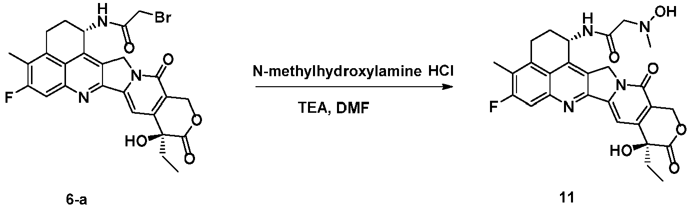

In a 10 mL vial, 1 mL of DMF was added, then N-methylhydroxyamine hydrochloride (30 mg, 0.36 mmol), TEA (58 mg, 0.57 mmol), and Compound 6-a (100 mg, 0.18 mmol) were added sequentially. The mixture was stirred to react for 1 h at room temperature. DMF was removed under reduced pressure, and the residue was separated and purified by silica gel column chromatography to afford Compound **11,** i.e., 2-N-methylhydroxyaminoacetyl exatecan (70 mg, yield 75%); ¹H NMR (500 MHz, DMSO-d₆) δ 8.44 (d, *J*=12.4 Hz, 1H), 7.79 (t, *J*=10.7 Hz, 1H), 7.30 (d, *J*=4.9 Hz, 1H), 6.63-6.41 (m, 1H), 5.59 (m, *J*=8.6, 5.0 Hz, 1H), 5.41 (d, *J=3.0* Hz, 2H), 5.32-5.12 (m, 2H), 3.17 (m, *J*=5.3 Hz, 2H), 2.73 (d, *J*=6.7 Hz, 3H), 2.39 (d, *J*=6.0 Hz, 3H), 2.24-2.08 (m, 2H), 1.85 (m, *J*=21.3, 7.1 Hz, 2H), 0.86 (t, *J*=7.3 Hz, 3H); LCMS: (M+H)⁺ 523.20 (calculated value: 522.19).

### Example 12: 2-ethoxyaminoacetyl exatecan (12)

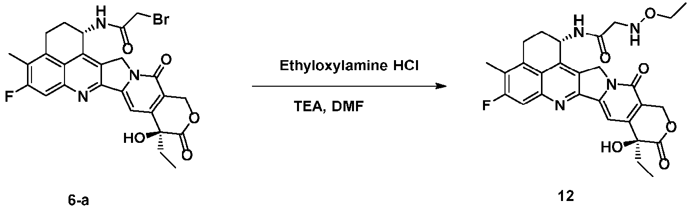

In a 10 mL vial, 1 mL of DMF was added, then ethoxy hydroxyamine hydrochloride (126 mg, 1.29 mmol), TEA (182 mg, 1.8 mmol), and Compound 6-a (100 mg, 0.18 mmol) were added sequentially. The mixture was stirred to react for 1 h at room temperature. DMF was removed under reduced pressure, and the residue was separated and purified by silica gel column chromatography to afford Compound **12,** i.e., 2-ethoxyaminoacetyl exatecan (81 mg, yield 83%); ¹H NMR (500 MHz, DMSO-d₆) δ 7.79 (m, *J*=11.0, 5.7 Hz, 1H), 7.31 (s, 1H), 5.63 (m, *J*=9.2, 5.4 Hz, 1H), 5.42 (s, 2H), 5.31-5.16 (m, 2H), 3.72-3.68 (m, 2H), 3.45-3.42 (m, 2H), 3.18 (m, *J*=17.3, 9.0 Hz, 2H), 2.40 (d, *J*=6.4 Hz, 3H), 2.27-2.10 (m, 2H), 1.86 (m, *J*=21.7, 14.6, 7.3 Hz, 2H), 1.24 (m, *J*=7.1 Hz, 1H), 1.00 (m, *J*=6.9 Hz, 2H), 0.87 (m, *J*=6.8 Hz, 3H); LCMS: (M+1)⁺ 537.10 (calculated value: 536.21).

### Example 13: 3-aminooxetane-3-acetyl exatecan (13)

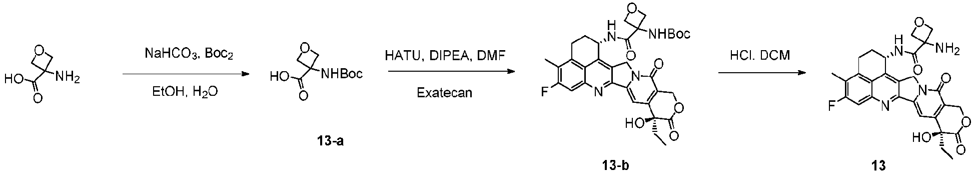

In a mixture of 10 mL of water and 10 mL of EtOH, 3-aminooxetane-3-carboxylic acid (0.5 g, 4.27 mmol) was added and the mixture was stirred until clear. Sodium bicarbonate (0.72 g, 8.54 mmol) was then added, followed by slowly dropwise adding a solution of Boc anhydride(0.98 g, 4.48 mmol) in EtOH (5 mL). After the addition was completed, the mixture was stirred to react for 2 h at room temperature. Ethanol was removed under reduced pressure, then hydrochloric acid was added dropwise to adjust the pH to approximately 4, and the product was extracted with DCM (100mL*3), dried over anhydrous sodium sulfate and concentrated to afford Compound 15-a, N-Boc-3-aminooxetane-3-carboxylic acid (0.8 g, yield 86%), LCMS: (M+1)⁺ 118.01 (calculated value: 117.10).

In 1 mL of DMF, exatecan mesylate (20 mg, 0.038 mmol, DIPEA (9.7 mg, 0.074 mmol), Compound **13-a** (16.5 mg, 0.075 mmol), and HATU (23.6 mg, 0.062 mmol) were added sequentially. The mixture was stirred to react for 6 h at room temperature. DMF was removed under reduced pressure, and the residue was separated and purified by silica gel column chromatography to afford Compound 15-b, N-Boc-3-aminooxetane-3-acetyl exatecan (15 mg, yield 65%), LCMS: (M+1)⁺ 635.20 (calculated value: 634.66).

Compound **13-b** (15 mg, 0.023 mmol) was dissolved in 2 mL of DCM, then a 1 mL solution of 4M hydrochloric acid in ethyl acetate was added, and the mixture was stirred to react for 30 min at room temperature, then concentrated to afford Compound **13,** i.e., 3-aminooxetane-3-acetyl exatecan (61 mg, yield 100%); ¹H NMR (500 MHz, DMSO-d₆) δ 8.55 (m,2H), 8.10 (d, *J*=8.8 Hz, 1H), 7.76 (m, 1H), 7.30 (d, *J*=7.1 Hz, 1H), 5.60-5.49 (m, 1H), 5.42 (m, 2H), 5.00-4.95 (m, 1H), 4.88 (d, *J*=6.2 Hz, 1H), 4.57 (d, *J*=6.3 Hz, 1H), 4.51 (d, *J*=6.2 Hz, 1H), 3.62 (m, 1H), 3.46 (m, 1H), 3.23 (m, 1H), 3.16-3.11 (m, 2H), 2.38 (m, 3H), 2.25-2.13 (m, 2H), 1.92-1.78 (m, 2H), 0.87 (m, 3H); LCMS: (M+1)⁺ 535.31 (calculated value: 534.54).

### Example 14: mc-Val-Cit-pab-formyl-2-N-methyl-N-aminoacetyl exatecan (DC-1)

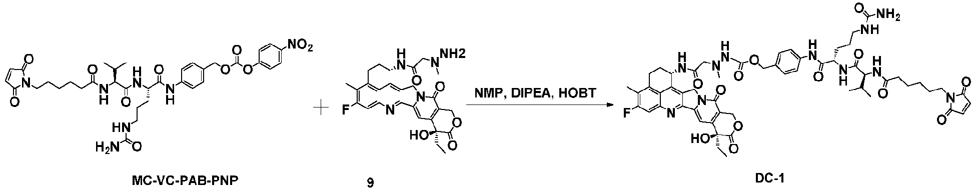

In 0.3 mL of NMP, Compound **9** (14.14 mg, 0.027 mmol), DIPEA (17.52 mg, 0.135 mmol), mc-Val-Cit-pab-PNP (20 mg, 0.027 mmol), and HOBt (7.33 mg, 0.054 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 10% to 55% acetonitrile in water for 40 min) and lyophilised to afford the Compound **DC-1,** i.e., mc-Val-Cit-pab-formyl-2-N-methyl-N-aminoacetyl exatecan (15.2 mg, 50% yield) , yellow solid; LCMS: (M+1)⁺ 1121.09 (calculated value: 1120.21).

### Example 15: mc-Val-Cit-pab-formyl-2-N-methyl-N-methylaminoacetyl exatecan (DC-2)

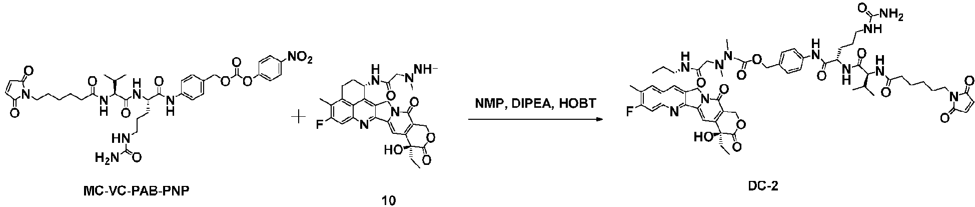

In 0.3 mL of NMP, Compound **10** (14.52 mg, 0.027 mmol), DIPEA (10.51 mg, 0.081 mmol), mc-Val-Cit-pab-PNP (20 mg, 0.027 mmol), and HOBt (7.33 mg, 0.054 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 10% to 55% acetonitrile in water for 40 min) and lyophilised to afford the Compound **DC-2,** i.e., mc-Val-Cit-pab-formyl-2-N-methyl-N-methylaminoacetyl exatecan (12.2 mg, 40% yield), yellow solid; LCMS: (M+1)⁺ 1135.02 (calculated value: 1134.23).

### Example 16: mc-Val-Cit-pab-formyl-exatecan-N-aminourea (DC-3)

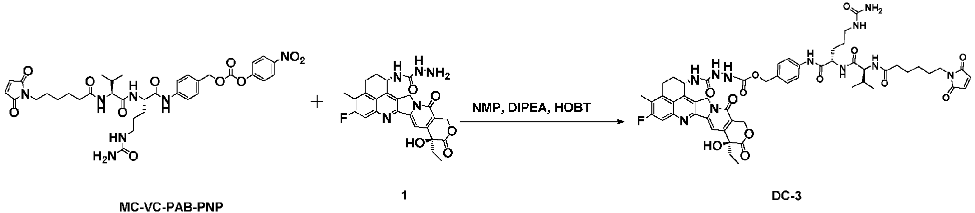

In 0.3 mL of NMP, Compound **1** (13.38 mg, 0.027 mmol), DIPEA (10.51 mg, 0.081 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water containing 0.1% TFA), and then eluted by medium-pressure reversed-phase C18 chromatography (gradient: 10% to 55% acetonitrile in water for 40 min) and lyophilised to afford the Compound **DC-3,** i.e., mc-Val-Cit-pab-formyl-exatecan-N-aminourea (13 mg, 45% yield), yellow solid; LCMS: (M+1)⁺ 1093.03 (calculated value: 1092.15).

### Example 17: mc-Val-Cit-pab-formyl-exatecan-N-methyl-N-aminourea (DC-4)

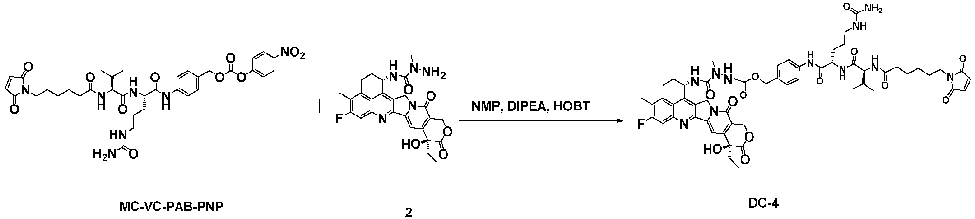

In 0.3 mL of NMP, Compound **2** (13.7 mg, 0.027 mmol), DIPEA (10.51 mg, 0.081 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 15% to 60% acetonitrile in water for 40 min) and lyophilised to afford the Compound **DC-4,** i.e., mc-Val-Cit-pab-formyl-exatecan-N-methyl-N-aminourea (9 mg, 30% yield), yellow solid; LCMS: (M+1)⁺ 1107.11 (calculated value: 1106.18).

### Example 18: mc-Val-Cit-pab-formyl-exatecan-N',N-dimethylaminourea (DC-5)

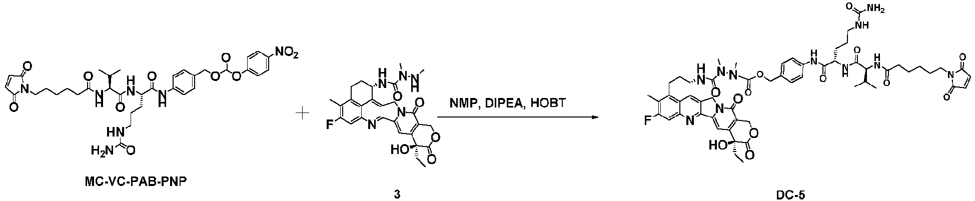

In 0.3 mL of NMP, Compound **3** (14.11 mg, 0.027 mmol), DIPEA (10.51 mg, 0.081 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 15% to 60% acetonitrile in water for 40 min) and lyophilised to afford the Compound **DC-5,** i.e., mc-Val-Cit-pab-formyl-exatecan-N',N-dimethylaminourea (12.1 mg, 40% yield), yellow solid; LCMS: (M+1)⁺ 1121.02 (calculated value: 1120.21).

### Example 19: mc-Val-Cit-pab-formyl-2-aminooxyacetyl exatecan (DC-6)

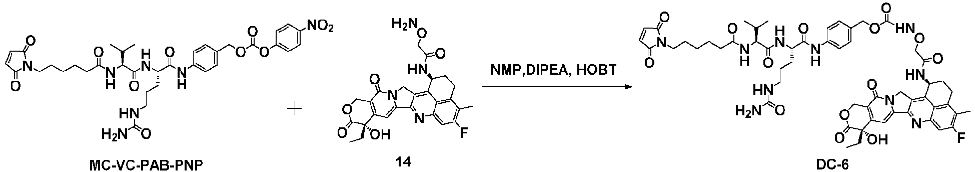

In 2 mL of DMF, exatecan mesylate (100 mg, 0.19 mmol), DIPEA (48.58 mg, 0.37 mmol), tertbutoxycarbonyl aminooxyacetic acid (45 mg, 0.23 mmol), and HATU (118 mg, 0.31 mmol) were added sequentially. The mixture was stirred to react for 4 h at room temperature. DMF was removed under reduced pressure, and the residue was purified by silica gel column chromatography to afford intermediate compound 2-Boc-aminooxyacetyl exatecan (95 mg, yield 83%); ¹H NMR (500 MHz, DMSO-d₆) δ 10.40 (s, 1H), 8.73 (d, *J*=8.6 Hz, 1H), 7.75 (d, *J*=10.8 Hz, 1H), 7.28 (d, *J*=2.1 Hz, 1H), 6.52 (d, *J*=1.5 Hz, 1H), 5.62 (dt, *J*=8.8, 4.4 Hz, 1H), 5.40 (d, *J*=3.3 Hz, 2H), 5.23 (d, *J*=17.9 Hz, 1H), 5.19-5.11 (m, 1H), 4.32 (d, *J*=15.8 Hz, 1H), 4.23 (d, *J*=15.9 Hz, 1H), 3.23-3.15 (m, 2H), 2.37 (d, *J*=6.5 Hz, 3H), 2.24 (dq, *J*=14.1, 4.9 Hz, 1H), 2.17 (tt, *J*=8.3, 5.1 Hz, 1H), 1.84 (ddp, *J*=21.4, 14.3, 7.2 Hz, 2H), 1.15 (s, 9H), 0.85 (t, *J*=7.2 Hz, 3H); LCMS: (M+1)⁺ 609.30 (calculated value: 608.18).

Compound 2-Boc-aminooxyacetyl exatecan (95 mg, 0.16 mmol) was dissolved in 1 mL of DCM, then a 1 mL solution of 4M hydrochloric acid in ethyl acetate was added, and the mixture was stirred to react for 30 min at room temperature, then concentrated to afford Compound **14,** i.e., 2-aminooxyacetyl exatecan (85 mg, yield 100%); ¹H NMR (500 MHz, DMSO-d₆) δ8.93 (d, *J*=8.5 Hz, 1H), 7.79 (d, *J*=10.5 Hz, 1H), 7.30 (s, 1H), 5.60 (dt, *J*=8.3, 4.1 Hz, 1H), 5.42 (s, 2H), 5.29 (s, 2H), 4.73-4.64 (m, 2H), 3.18 (s, 2H), 2.85 (s, 3H), 2.39 (s, 3H), 2.25 (dt, *J*=8.8, 4.6 Hz, 1H), 2.19-2.10 (m, 1H), 1.85 (dp, *J*=21.4, 7.2 Hz, 2H), 0.86 (t, *J*=7.3 Hz, 3H); LCMS: (M+1)⁺ 509.20 (calculated value: 508.18).

In 0.7 mL of NMP, Compound **14** (32.17 mg, 0.063 mmol), DIPEA (24.53 mg, 0.189 mmol), mc-Val-Cit-pab-PNP (70 mg, 0.095 mmol), and HOBt (8.55 mg, 0.063 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 10% to 55% acetonitrile in water for 40 min) and lyophilised to afford the Compound **DC-6,** i.e., mc- Val-Cit-pab-formyl-2-aminooxyacetyl exatecan (31.5 mg, 31% yield) , yellow solid; LCMS: (M+1)⁺ 1108.10 (calculated value: 1107.16).

### Example 20: mc-Val-Cit-pab-formyl-N-methylaminooxyacetyl exatecan (DC-7)

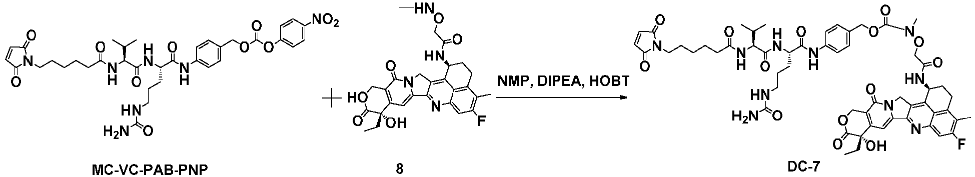

In 0.7 mL of NMP, Compound **8** (33.05 mg, 0.063 mmol), DIPEA (24.53 mg, 0.189 mmol), mc-Val-Cit-pab-PNP (70 mg, 0.095 mmol), and HOBt (8.55 mg, 0.063 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 10% to 55% acetonitrile in water for 40 min) and lyophilised to afford the Compound **DC-7,** i.e., mc-Val-Cit-pab-formyl-2-methoxyaminoacetyl exatecan (42 mg, 39% yield) , yellow solid; LCMS: (M+1)⁺ 1122.05 (calculated value: 1121.09).

### Example 21: mc-Val-Cit-pab-formyl-exatecan-N'-aminooxalyldiamine (DC-8)

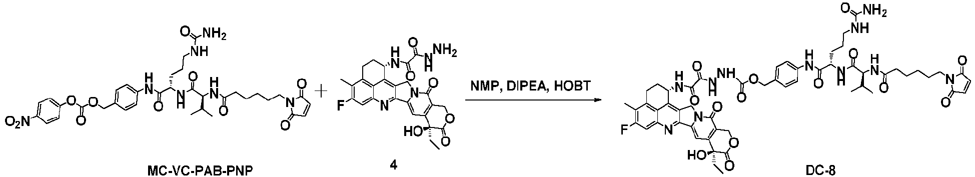

In 0.3 mL of NMP, Compound **4** (14.14 mg, 0.027 mmol), DIPEA (17.52 mg, 0.135 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 10% to 55% acetonitrile in water for 40 min) and lyophilised to afford the Compound **DC-8,** i.e., mc-Val-Cit-pab-formyl-exatecan-N'-aminooxalyldiamine (13 mg, 45% yield), yellow solid; LCMS: (M+1)⁺ 1121.02 (calculated value: 1120.16).

### Example 22: mc-Val-Cit-pab-formyl-exatecan N'-amino-N-methyloxalyldiamine (DC-9)

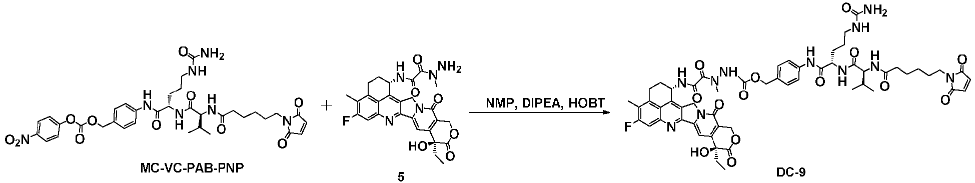

In 0.3 mL of NMP, Compound **5** (14.52 mg, 0.027 mmol), DIPEA (17.52 mg, 0.135 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 10% to 55% acetonitrile in water for 40 min) and lyophilised to afford the Compound **DC-9,** i.e., mc-Val-Cit-pab-formyl-exatecan N'-amino-N-methyloxalyldiamine (13 mg, 45% yield), yellow solid; LCMS: (M+1)⁺ 1135.10 (calculated value: 1134.19).

### Example 23: mc-Val-Cit-pab-formyl-N-hydroxyaminoacetyl exatecan (DC-10)

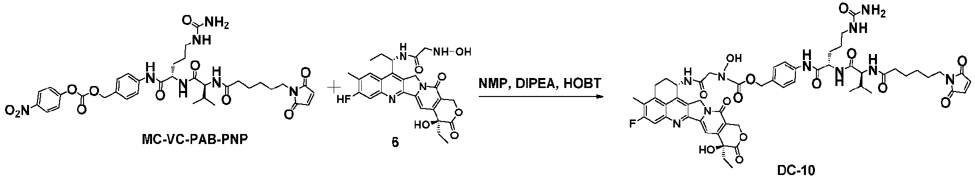

In 0.3 mL of NMP, Compound **6** (13.79 mg, 0.027 mmol), DIPEA (10.51 mg, 0.081 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 5% to 50% acetonitrile in water over 40 min) and lyophilised to afford the Compound **DC-10,** i.e., mc-Val-Cit-pab-formyl-N-hydroxyaminoacetyl exatecan (13 mg, 45% yield) , yellow solid; LCMS: (M+1)⁺ 1108. 03 (calculated value: 1107.16).

### Example 24: mc-Val-Cit-pab-formyl-N-methoxyaminoacetyl exatecan (DC-11)

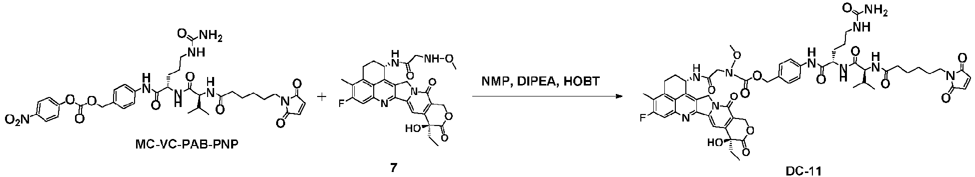

In 0.3 mL of NMP, Compound 7 (14.17 mg, 0.027 mmol), DIPEA (10.51 mg, 0.081 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 15% to 50% acetonitrile in water over 40 min) and lyophilised to afford the Compound **DC-11,** i.e., mc-Val-Cit-pab-formyl-N-methoxyaminoacetyl exatecan (13 mg, 45% yield) , yellow solid; LCMS: (M+1)⁺ 1122.06 (calculated value: 1121.19).

### Example 25: mc-Val-Cit-pab-formyl-N-ethoxyaminoacetyl exatecan (DC-12)

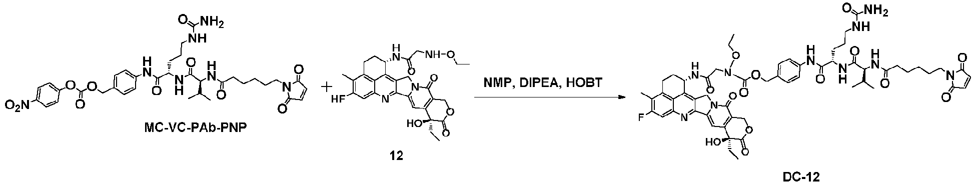

In 0.3 mL of NMP, Compound **12** (14.17 mg, 0.027 mmol), DIPEA (10.51 mg, 0.081 mmol), mc-Val-Cit-pab-PNP (20 mg, 0.027 mmol), and HOBt (3.66 mg, 0.027 mmol) were added sequentially. The mixture was reacted for 1 h at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 15% to 50% acetonitrile in water over 40 min) and lyophilised to afford the Compound **DC-12,** i.e., mc-Val-Cit-pab-formyl-N-ethoxyaminoacetyl exatecan (13 mg, 45% yield) , yellow solid; LCMS: (M+1)⁺ 1136.02 (calculated value: 1135.20).

### Example 26: mc-Gly-Gly-Phe-Gly-methoxy-N-methylaminoacetyl exatecan (DC-13)

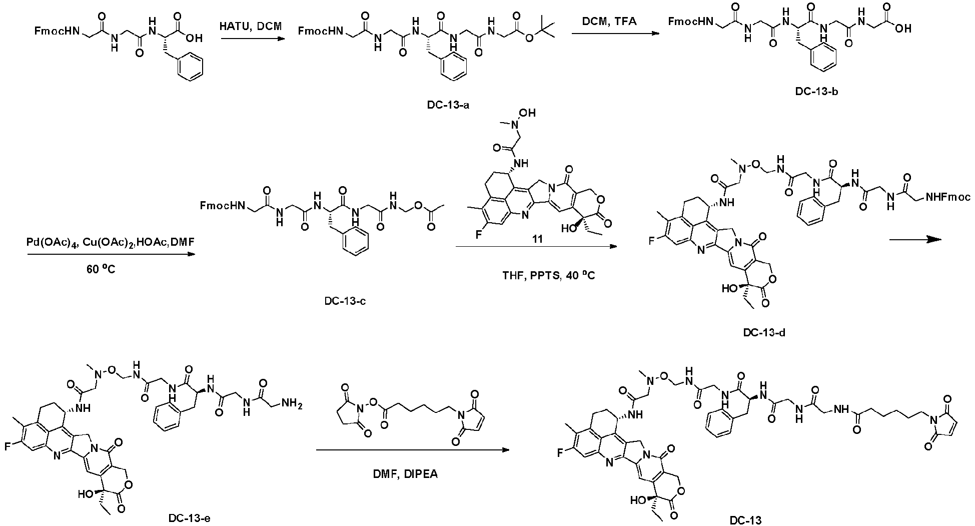

Fmoc-Gly-Gly-Phe-OH (10 g, 19.9 mmol) was taken, and 200 mL of DCM was added. Then, HATU (11.43 g, 29.85 mmol), DIPEA (5.13 g, 39.8 mmol), and Gly-Gly-OtBu (4.11 g, 21.89 mmol) were added. The mixture was reacted for 15 h at room temperature. Then, 200 mL of DCM was added to dilute the mixture, which was then washed with a saturated solution of sodium bicarbonate, dried over anhydrous sodium sulfate, and purified by silica gel column chromatography to afford white solid **DC-13-a,** Fmoc-Gly-Gly-Phe-Gly-Gly-OtBu (10.7 g, 79%); LCMS: (M+1)⁺ 672.5 (calculated value: 671.75).

**DC-13-a** (10.7 g, 15.9 mmol) was taken, and 110 mL of DCM and 50 mL of TFA were added. The mixture was reacted for 15 h at room temperature then concentrated, and 100 mL of EA was added. The mixture was filtered, and the filter cake was dried in an oven at 40°C to afford an off-white solid **DC-13-b,** Fmoc-Gly-Gly-Phe-Gly-Gly (8.8 g, 90%); LCMS: (M+1)⁺ 616.10(calculated value: 615.23).

**DC-13-b** (3 g, 4.8 mmol) was taken and dissolved in 30 mL of DMF, stirred to dissolve. Copper(II) acetate (1.46 mmol, 0.26 g), acetic acid (9.7 mmol, 0.58 g), and lead tetraacetate (5.3 mmol, 2.38 g) were added sequentially. The mixture was heated to 60°C and reacted for 20 min. The reaction mixture was then poured into ice water, and ethyl acetate was added for extraction. After drying over anhydrous sodium sulfate, the reaction mixture was concentrated to afford an off-white solid product **DC-13-c,** Fmoc-Gly-Gly-Phe-Gly-N-hydroxymethylacetate (1.81 g, 59%); LCMS: (M+1)⁺ 630.10 (calculated value: 629.25).

**DC-13-c** (0.05 g, 0.076 mmol) was taken, and 5 mL of THF was added. Then, Compound 11 (0.04 g, 0.076 mmol) and PPTS (3.8 mg, 0.015 mmol) were added. The mixture was refluxed at 60°C to react for 16 h under argon protection and purified by silica gel column chromatography to afford a white solid **DC-13-d,** Fmoc-Gly-Gly-Phe-Gly-methoxy-N-methylaminoacetyl exatecan (43 mg, 50%); LCMS: (M+1)⁺ 1093.05 (calculated value: 1092.15).

DC-13-d (43 mg, 0.039 mmol) was dissolved in 0.5 mL of DMF, piperidine (33 mg, 0.39 mmol) was added, and the mixture was stirred to react for 1 h at room temperature. The reaction mixture was concentrated to afford a yellow-brown solid **DC-13-e,** Gly-Gly-Phe-Gly-methoxy-N-methylaminoacetyl exatecan (34 mg, 100%); LCMS: (M+1)⁺ 871.02 (calculated value: 869.91).

**DC-13-e** (34 mg, 0.039 mmol) was dissolved in 0.5 mL of anhydrous DMF. Then, succinimidyl 6-(maleimido)hexanoate (12 mg, 0.039 mmol) and DIPEA (5 mg, 0.039 mmol) were added sequentially. The mixture was reacted for 30 min at room temperature. The reaction mixture was injected into a 25 g C18 pre-column (first equilibrated with acetonitrile, then with water, the aqueous phase containing 0.1% TFA), and then eluted by medium-pressure reversed-phase C18 chromatography (gradient: 15% to 50% acetonitrile in water for 40 min) and lyophilised to afford the Compound **DC-13,** mc-Gly-Gly-Phe-Gly-methoxy-N-methylaminoacetyl exatecan (12.4 mg, 30% yield), yellow solid; LCMS: (M+1)⁺ 1064.05 (calculated value: 1063.11).

### Example 27: 4-(maleimidohexyloxy)benzaldehyde-aminooxyacetyl exatecan oxime (DC-14)

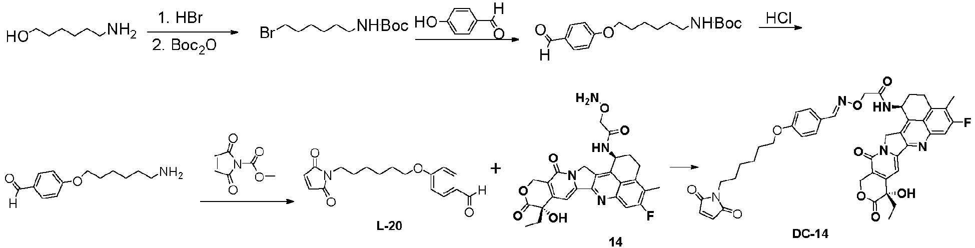

In a 100 mL single-neck flask, 6-amino-1-hexanol (6.0 g, 51.20 mmol), and 60 mL of 48% aqueous HBr solution were added. The mixture was refluxed and stirred to react at 90°C for 18 h. The pH of the reaction mixture was adjusted to 8-9 with solid sodium bicarbonate in an ice bath, then Boc anhydride (11.2 g, 51.20 mmol) was added, and the mixture was slowly heated to room temperature and stirred overnight. The reaction mixture was filtered, and the filtrate was extracted with EtOAc to afford the target product. The organic phase was washed three times with a 5% NaCl solution and then dried with anhydrous Na₂SO₄. The dried organic phase was filtered and concentrated to afford an off-white solid product, N-Boc-6-bromohexanamine (10.0 g, 71.4% yield); LCMS: (M+1)⁺ 281.12 (calculated value: 280.21).

In a 100 mL single-neck flask, 50 mL of acetonitrile was added, and N-Boc-6-bromohexanamine (5.0 g, 17.84 mmol), p-hydroxybenzaldehyde (2.2 g, 17.84 mmol), and anhydrous potassium carbonate (6.2 g, 44.61 mmol) were added sequentially. The mixture was refluxed and stirred to react at 60°C for 18 h. The reaction mixture was filtered, and 10.0 g of silica gel sand was added to the filtrate. The product was purified by column chromatography using PE and EA, and concentrated to afford an off-white solid product, 4-(N-Boc-hexyloxy)benzaldehyde (5.0 g, 87.7% yield); LCMS: (M+1)⁺ 322.14 (calculated value: 321.42).

In a 100 mL single-neck flask, 3 mL of 1,4-dioxane was added, then 4-(N-Boc-hexyloxy)benzaldehyde (1.0 g, 4.52 mmol) and 3 mL of 4 M HCl/1,4-dioxane solution were added sequentially. The mixture was stirred to react for 3 h at room temperature. The pH of the reaction mixture was adjusted to 9 with a saturated solution of sodium bicarbonate in an ice bath. Then, N-methoxycarbonylmaleimide (700.90 mg, 4.52 mmol) was added, and the mixture was stirred to react for 2 h in an ice bath. The solid was filtered out, and the filter cake was washed with 10 mL of purified water. The filter cake was dried at 35°C to afford an off-white solid product **L-20** (1.0 g, 73.5% yield); LCMS: (M+1)⁺ 302.23 (calculated value: 301.34).

In 3 mL of methanol, L-20 (24.59 mg, 0.081 mmol), pyridine (14.2 mg, 0.179 mmol), and anhydrous sodium sulfate (23.18 mg, 0.163 mmol) were added, followed by the addition of Compound **14** (83 mg, 0.163 mmol). The mixture was stirred to react for 1 h at room temperature. After the reaction was completed, the reaction mixture was added into 1 g silica gel column chromatography to afford the product **DC-14,** 4-(maleimidohexyloxy)benzaldehyde-aminooxyacetyl exatecan oxime (36 mg, 56% yield); LCMS: (M+1)⁺ 792.11 (calculated value: 791.30).

### Example 28: 4-(maleimidoethyloxyethoxy)benzaldehyde-aminooxyacetyl exatecan oxime (DC-15)

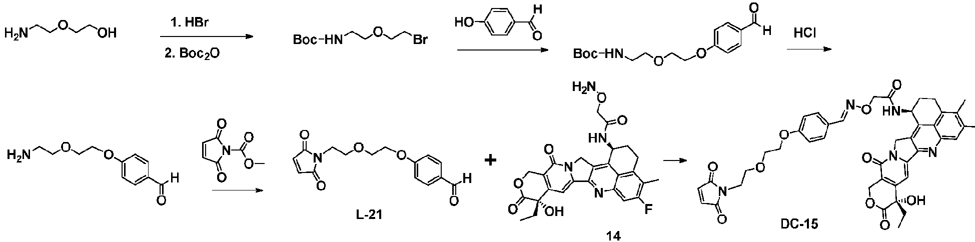

In a 100 mL single-neck flask, diglycolamine (6.0 g, 57.07 mmol), and 60 mL of 48% aqueous HBr solution were added sequentially. The mixture was refluxed and stirred to react at 90°C for 18 h. The pH of the reaction mixture was adjusted to between 8 and 9 with solid sodium bicarbonate in an ice bath. Boc anhydride (12.46 g, 57.07 mmol) was added, and the mixture was slowly heated to room temperature and stirred overnight. The reaction mixture was filtered, and the filtrate was extracted with EtOAc to afford the target product. The organic phase was washed three times with a 5% NaCl solution and then dried with anhydrous Na₂SO₄. The dried organic phase was filtered, concentrated to afford an off-white solid product, N-Boc-bromoethoxyethylamine (10.8g, yield 70.6%); LCMS: (M+1)⁺ 269.02 (calculated value: 268.15). In a 100 mL single-neck flask, 50 mL of acetonitrile was added, N-Boc-bromoethoxyethylamine (5.0 g, 18.65 mmol), p-hydroxybenzaldehyde (2.28 g, 18.65 mmol), and anhydrous potassium carbonate (6.44 g, 46.62 mmol) were added sequentially. The mixture was refluxed and stirred to react at 60°C for 18 h. The reaction mixture was filtered, and 10.0 g of silica gel sand was added. The product was purified by column chromatography using PE and EA, and concentrated to afford an off-white solid product, 4-(N-Boc-aminoethoxyethoxy)benzaldehyde (5.3 g, yield 91.8%); LCMS: (M+1)⁺ 310.24 (calculated value: 309.36).

In a 100 mL single-neck flask, 3 mL of 1,4-dioxane was added, then 4-(N-Boc-aminoethoxyethoxy)benzaldehyde (0.6 g, 1.94 mmol), and 3 mL of 4 M HCl/1,4-dioxane solution were added sequentially. The mixture was stirred for 3 h at room temperature. The pH of the reaction mixture was adjusted to 9 with a saturated solution of sodium bicarbonate in an ice bath. N-methoxycarbonylmaleimide (300.83 mg, 1.94 mmol) was added, and the mixture was stirred to react for 2 h under an ice bath. The solid was filtered out, and the filter cake was washed with 10 mL of purified water. The filter cake was dried at 35 °C to afford an off-white solid product **L-021** (500 mg, yield 89%); LCMS: (M+1)⁺ 290.13 (calculated value: 289.29).

In 3 mL of methanol, L-021 (23.61 mg, 0.082 mmol), pyridine (14.2 mg, 0.179 mmol), and anhydrous sodium sulfate (23.18 mg, 0.163 mmol) were added, followed by the addition of Compound **14** (83 mg, 0.163 mmol). The mixture was stirred to react for 1 h at room temperature. After the reaction was completed, the reaction mixture was added into 1 g silica gel column chromatography to afford the product **DC-15,** 4-(maleimidoethyloxyethoxy)benzaldehyde-aminooxyacetyl exatecan oxime (42 mg, yield 66%); LCMS: (M+1)⁺ 780.12 (calculated value: 779.26).

### Example 29: HS627-succinimidyl-N-hexanoyl-Val-Cit-pab-formyl-exatecan-N-aminourea (ADC-3a)

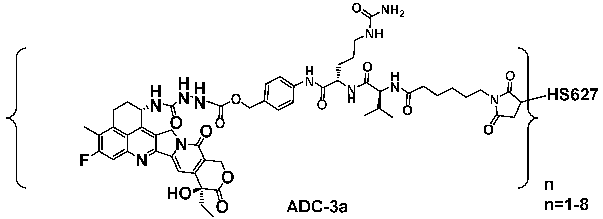

The HS627 antibody (20.0 mg/mL, 10 mg, 0.066 mmol) was taken and the pH was adjusted to 7.2 with 1 M Na₂HPO₄ solution, followed by the addition of 0.1 M disodium ethylenediaminetetraacetic acid solution (25 µL), and the addition of formulated TCEP-HCl (tris(2-carboxyethyl)phosphine hydrochloride) solution (10 mM, 0.04 mL), and the mixture was reacted by spinning disc for 90 min at room temperature 25 °C.

**DC-3** (0.9 mg, 0.8 mmol) was dissolved in 0.09 mL of DMA, then added to the above solution system, mixed well, and the mixture was reacted by spinning disc for 2h at room temperature. After the reaction was completed, the buffer was replaced by a NAP-5 gel column (Cytiva) with 20 mM L-histidine acetate buffer, pH 6.0, 120 mM sucrose, 0.2 g/L polysorbate 20, to afford **ADC-3a** (1.8 mg/mL, 3 mL).
UV-HPLC calculation average: n=7.40

### Example 30: HS627-succinimidyl-N-hexanoyl-Val-Cit-pab-formyl-2-aminooxyacetyl exatecan (ADC-6a)

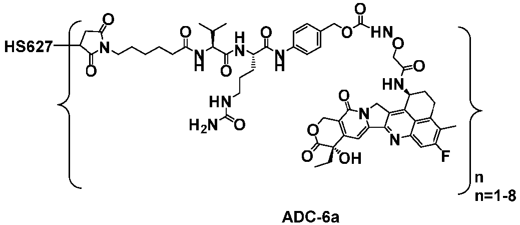

The HS627 antibody (20.0 mg/mL, 10 mg, 0.066 mmol) was taken and the pH was adjusted to 7.2 with 1 M Na₂HPO₄ solution, followed by the addition of 0.1 M disodium ethylenediaminetetraacetic acid solution (25 µL), and the addition of formulated TCEP-HCl (tris(2-carboxyethyl)phosphine hydrochloride) solution (10 mM, 0.04 mL), and the mixture was reacted by spinning disc for 90 min at room temperature 25 °C.

**DC-6** (0.9 mg, 0.8 mmol) was dissolved in 0.09 mL of DMA, then added to the above solution system, mixed well, and the mixture was reacted by spinning disc for 2h at room temperature. After the reaction was completed, the buffer was replaced by a NAP-5 gel column (Cytiva) with 20 mM L-histidine acetate buffer, pH 6.0, 120 mM sucrose, 0.2 g/L polysorbate 20 to afford **ADC-6a** (3.2 mg/mL, 2 mL).
UV-HPLC calculation average: n=7.70

### Example 31: HS627-succinimidyl-N-hexanoyl-Val-Cit-pab-formyl-N-methylaminooxyacetyl exatecan (ADC-7a)

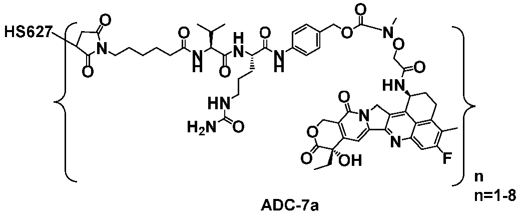

The HS627 antibody (20.0 mg/mL, 10 mg, 0.066 mmol) was taken and the pH was adjusted to 7.2 with 1 M Na₂HPO₄ solution, followed by the addition of 0.1 M disodium ethylenediaminetetraacetic acid solution (25 µL), and the addition of formulated TCEP-HCl (tris(2-carboxyethyl)phosphine hydrochloride) solution (10 mM, 0.04 mL), and the mixture was reacted by spinning disc for 90 min at room temperature 25 °C.

**DC-7** (0.9 mg, 0.8 mmol) was dissolved in 0.09 mL of DMA, added to the above solution system, mixed well, and the mixture was reacted by spinning disc for 2h at room temperature. After the reaction was completed, the buffer was replaced by a NAP-5 gel column (Cytiva) with 20 mM L-histidine acetate buffer, pH 6.0, 120 mM sucrose, 0.2 g/L polysorbate 20 to afford **ADC-7a** (3.1 mg/mL, 2 mL).
UV-HPLC calculation average: n=7.30

### Example 32: HS627-succinimidyl-4-(N-hexyloxy)benzaldehyde oxime oxyacetyl exatecan (ADC-14a)

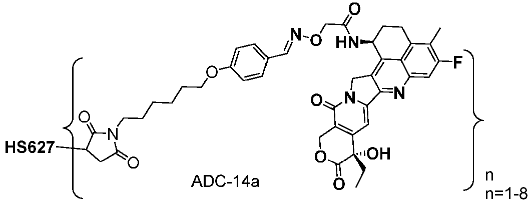

The HS627 antibody (20.0 mg/mL, 10 mg, 0.066 mmol) was taken and the pH was adjusted to 7.2 with 1 M Na₂HPO₄ solution, followed by the addition of 0.1 M disodium ethylenediaminetetraacetic acid solution (25 µL), and the addition of formulated TCEP-HCl (tris(2-carboxyethyl)phosphine hydrochloride) solution (10 mM, 0.04 mL), and the mixture was reacted by spinning disc for 90 min at room temperature 25 °C.

**DC-14** (0.63 mg, 0.8 mmol) was dissolved in 0.063 mL of DMA, then added to the above solution system, mixed well, and the mixture was reacted by spinning disc for 2h at room temperature. After the reaction was completed, the buffer was replaced with a NAP-5 gel column (Cytiva) with 20 mM L-histidine acetate buffer, pH 6.0, 120 mM sucrose, 0.2 g/L polysorbate 20 to afford **ADC-14a** (2.9 mg/mL, 2 mL).

UV-HPLC calculation average: n=4.3.

### Example 33: HS627-succinimidyl-4-(N-ethoxyethoxy)benzaldehyde oxime oxoacetyl exatecan (ADC-15a)

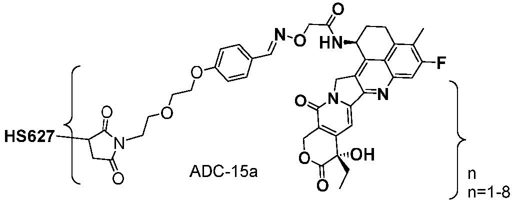

The HS627 antibody (20.0 mg/mL, 10 mg, 0.066 mmol) was taken and the pH was adjusted to 7.2 with 1 M Na₂HPO₄ solution, followed by the addition of 0.1 M disodium ethylenediaminetetraacetic acid solution (25 µL), and the addition of formulated TCEP-HCl (tris(2-carboxyethyl)phosphine hydrochloride) solution (10 mM, 0.04 mL), and the mixture was reacted by spinning disc for 90 min at room temperature 25 °C.

**DC-15** (0.62 mg, 0.8 mmol) was dissolved in 0.062 mL of DMA, then added to the above solution system, mixed well, and the mixture was reacted by spinning disc for 2h at room temperature. After the reaction was completed, the buffer was replaced with a NAP-5 gel column (Cytiva) to 20 mM L-histidine acetate buffer, pH 6.0, 120 mM sucrose, 0.2 g/L polysorbate 20 to afford **ADC-15a** (3.2 mg/mL, 2 mL).

UV-HPLC calculation average: n=7.6.

### Effect Example 1: Activity for inhibiting tumour cell growth

### Test method for in vitro inhibitory activity of compounds (small molecule toxins)

Human esophageal cancer cells OE33, and human breast adenocarcinoma cells SK-BR-3 were cultured in RPMI1640 (Cellmax) containing 10% fetal bovine serum (Cellmax), respectively. Cells in the exponential growth phase were diluted to a concentration of 1×10⁵cells/mL by culture medium and added to 96-well cell culture plates at 100µL per well, then incubated overnight in a 37°C incubator with 5% CO₂. On the following day, the small molecules were diluted with culture medium to 10,000 nM, 2,000 nM, 400 nM, 80 nM, 16 nM, 3.2 nM, 0.64 nM, 0.13 nM, respectively, and the diluted compounds were added to 96-well cell culture plates at 2 µL per well, with three replicate wells set-up for each concentration, and for the negative and blank control groups, in which the compounds had not been added, 2 µL of dilution was added per well. After the addition, the plates were returned to the 37°C incubator with 5% CO₂ to continue incubation for 72 h. After incubation, the cell culture plates were removed, and the culture medium was aspirated using a pipette. Then, 100µL of culture medium containing 10% CCK-8 was added to each well, and the plates were incubated at 37°C for 3 hours. After incubation, the plates were removed, kept in the dark, and placed in ELISAplate. Absorbance was measured at 630nm as the reference wavelength and 450nm as the test wavelength. The IC₅₀ values (Tables 1-3) were calculated using the four-parameter logistic regression method in GraphPad, based on the absorbance values. DNA topoisomerase I inhibitor Dxd was used as positive control drug. For the IC₅₀ values, wherein "++++" indicates an IC₅₀ < 50 nM; "+++" indicates an IC₅₀ between 50 nM and 100 nM; "++" indicates an IC₅₀ between 100 nM and 500 nM; and "+" indicates an IC₅₀ > 500 nM.

### Test method for in vitro inhibitory activity of ADC

Human esophageal cancer cells OE33, lung cancer cells NCI-H1975 and breast cancer cells MDA-MB-231, which were used for activity testing, were cultured to the exponential growth phase in RPMI1640 (Cellmax), RPMI1640 (Cellmax) and DMEM (Cellmax) medium containing 10% fetal bovine serum (Cellmax), respectively. After trypsin digestion, the supernatant was centrifuged and discarded, and the cells were diluted with medium to 3×10⁴cells/mL, 0.5×10⁴cells/mL and 1.5×10⁴cells/mL, respectively, and then added into 96-well cell culture plates at 100 µL per well, and returned to incubate overnight in a 37°C incubator with 5% CO₂. On the following day, the ADC to be measured was diluted with culture medium to 2000 nM, 400 nM, 80 nM, 16 nM, 3.2 nM, 0.64 nM, 0.128 nM, 0.026 nM, and the diluted ADC was added to 96-well cell culture plates at 100 µL per well, with three replicate wells set-up for each concentration, and for the negative and blank control groups, in which the ADC had not been added, 100 µL of culture medium was added per well. After the addition, the plates were returned to the 37°C incubator with 5% CO₂ to continue incubation for 6 days. After incubation, the cell culture plates were removed, and the culture medium was aspirated using a pipette. Then, 100µL of culture medium containing 10% CCK-8 was added to each well, and the plates were incubated at 37°C for 3 hours. After incubation, the plates were removed, kept in the dark, and placed in ELISA plate. Absorbance was measured at 630nm as the reference wavelength and 450nm as the test wavelength. The IC₅₀ values (Tables 1 and 3) were calculated using the four-parameter logistic regression method in GraphPad, based on the absorbance values. ADC drug DS-8201a was used as positive control drug. For the IC₅₀ values, wherein "++++" indicates an IC₅₀ < 50 nM; "+++" indicates an IC₅₀ between 50 nM and 100 nM; "++" indicates an IC₅₀ between 100 nM and 500 nM; and "+" indicates an IC₅₀ > 500 nM.

The structure of the positive control compound DXD is:

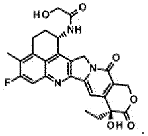

**Table 1. Inhibitory activity against OE33**

| Compound No. | IC₅₀/nM | Compound No. | IC₅₀/nM | ADC No. | IC₅₀/nM |
|---|---|---|---|---|---|
| 1 | +++ | 8 | ++++ | ADC-3a | ++ |
| 2 | ++++ | 9 | ++++ | ADC-6a | +++ |
| 3 | ++++ | 10 | +++ | ADC-7a | ++++ |
| 4 | +++ | 11 | +++ | ADC-1b | ++ |
| 5 | +++ | 12 | +++ | ADC-3b | + |
| 6 | +++ | 13 | ++++ | ADC-6b | ++++ |
| 7 | ++++ | Dxd | ++++ | ADC-7b | ++++ |
| DS-8201a | +++ | | | | |

**Table 2 Inhibitory activity against SK-BR-3**

| Compound No. | IC₅₀/nM | Compound No. | IC₅₀/nM | Compound No. | IC₅₀/nM |
|---|---|---|---|---|---|
| 1 | +++ | 5 | ++++ | 13 | ++++ |
| 3 | ++++ | 8 | ++++ | Dxd | ++++ |
| 4 | +++ | | | | |

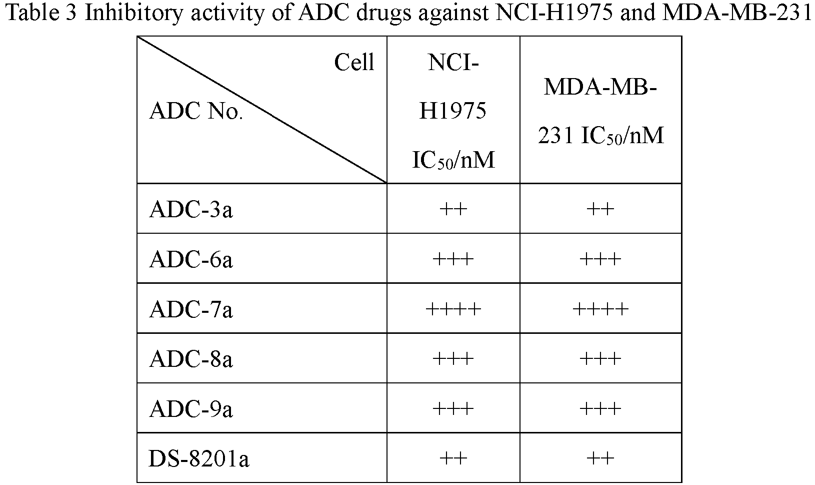

The compounds of the Examples provided by the present disclosure all have a good inhibitory effect on the growth of cancer cells such as esophageal cancer cells and breast adenocarcinoma cells , and the IC₅₀ values of most compounds such as compounds 2, 3, 7, 8, 9 and 13 for inhibiting the growth of esophageal cancer cells are all lower than 10nM, thus having significant anticancer activity.

The compounds of the Examples of the present disclosure are used to construct antibody-drug conjugates, and the resulting conjugates have significant inhibitory activities against esophageal cancer cells, lung cancer cells, and breast cancer cells, and the exemplary conjugates ADC-3a, ADC-6a, and ADC-7a have significant inhibitory activities against the above cells, and the inhibitory activity of ADC-7a is even lower than 10 nM.

## Claims

1. A compound represented by formula (I) or a pharmaceutically acceptable salt, a stereoisomer, or a prodrug thereof: 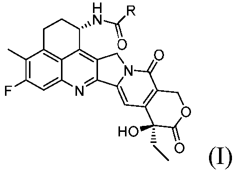 wherein,
R is selected from -Z-R₁, wherein, Z is a single bond or C=O;
R₁ is selected from -(CH₂)ₙNRₐR_{b}, -CH₂ORₐ, -NORₐ, -(CH₂)ₙONRₐR_{b} or -R₃; n is selected from 0, 1, 2 or 3;
Rₐ, R_{b} are each independently selected from hydrogen, hydroxy, amino, C₁-C₆ alkyl, C₁-C₆ alkylamino, aminoC₁-C₆ alkyl or C₁-C₆ alkoxy;
R₃ is selected from 3 to 6 membered cycloalkyl or 3 to 6 membered heterocycloalkyl containing N, O, or S heteroatoms, R₃ is optionally further substituted with R_{c};
R_{c} is selected from hydrogen, hydroxy, amino, C₁-C₆ alkyl, C₁-C₆ alkylamino or C₁-C₆ alkoxy; provided that -Z-R₁ is not -NH₂, -CH₂OH, -CH₂NH₂ or -CH₂OCH₂NH₂.

2. The compound or pharmaceutically acceptable salt, stereoisomer, or prodrug thereof according to claim 1, **characterized in that**, R₁ is selected from -NRₐR_{b}, -CH₂NRₐR_{b}, -CH₂ORₐ, -NORₐ, - (CH₂)ₙONRₐR_{b} or -R₃;
preferably, R₁ is selected from -NRₐR_{b}, -C(O)NRₐR_{b}, -CH₂NRₐR_{b} or -CH₂ONRₐR_{b} or -R₃;
preferably, Rₐ, R_{b} are each independently selected from hydrogen, hydroxy, amino, C₁-C₆ alkyl, C₁-C₆ alkylamino or C₁-C₆ alkoxy;
preferably, Rₐ, R_{b} are each independently selected from hydrogen, hydroxy, amino, methyl, methylamino or methoxy;
R₃ is selected from 3 to 6 membered heterocycloalkyl containing N, O, or S heteroatoms, R₃ is optionally further substituted with R_{c};
R_{c} is selected from hydrogen, hydroxy, amino, C₁-C₆ alkyl, C₁-C₆ alkylamino or C₁-C₆ alkoxy.

3. The compound or pharmaceutically acceptable salt, stereoisomer, or prodrug thereof according to claim 2, wherein, R₁ is selected from -NHNH₂, -N(CH₃)NH₂, -N(CH₃)NHCH₃, -C(O)NHNH₂, -C(O)N(CH₃)NH₂, -CH₂NHOH, -CH₂NHOCH₃, -CH₂ONH₂, -CH₂ONHCH₃, -CH₂NCH₃NH₂, - CH₂N(CH₃)NHCH₃, -CH₂N(CH₃)OH, -CH₂NHOCH₂CH₃, hydroxyoxetanyl, aminooxetanyl.

4. The compound or pharmaceutically acceptable salt, stereoisomer, or prodrug thereof according to any one of claims 1 to 3, the compound represented by formula (I) is the compound represented by formula (Ia), (Ib), (Ic) or formula (Id): 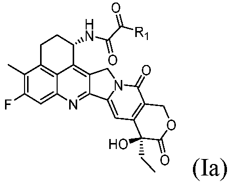
in formula (Ia), R₁ is defined the same as the compound represented by formula (I);
preferably, R₁ is -NHNH₂ or -N(CH₃)NH₂; 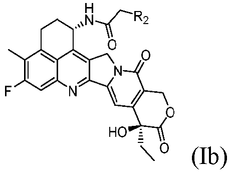
in formula (Ib), R₂ is selected from -NHOH, -ONH₂, -NHO(C₁-C₃ alkyl), -ONH(C₁-C₃ alkyl), - N(C₁-C₃ alkylamino), -N(C₁-C₃ alkyl)NH(C₁-C₃ alkyl), -N(C₁-C₃ alkyl)OH or -NHO(C₁-C₃ alkyl);
preferably, R₂ is selected from -NHOH, -NHOCH₃, -ONH₂, -ONHCH₃, -NCH₃NH₂, - N(CH₃)NHCH₃, -N(CH₃)OH or -NHOCH₂CH₃; 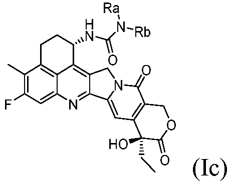
in formula (Ic), Rₐ, R_{b} are defined the same as the compound represented by formula (I); provided that Rₐ, R_{b} are not both H;
preferably, Rₐ, R_{b} are each independently selected from hydrogen, amino, C₁-C₃ alkyl, C₁-C₃ alkylamino;
preferably, -NRₐR_{b} is selected from -NHNH₂, -N(CH₃)NH₂ or -N(CH₃)NHCH₃; 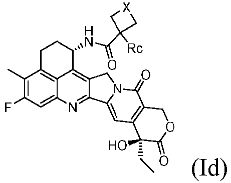
in formula (Id), X is CH₂, NH, O or S; preferably, X is CH₂ or O; more preferably, X is O;
R_{c} is selected from hydrogen, hydroxy, amino, C₁-C₆ alkyl, C₁-C₆ alkylamino or C₁-C₆ alkoxy; preferably, R_{c} is selected from hydroxy or amino.

5. The following compound or a pharmaceutically acceptable salt, a stereoisomer, or a prodrug thereof:

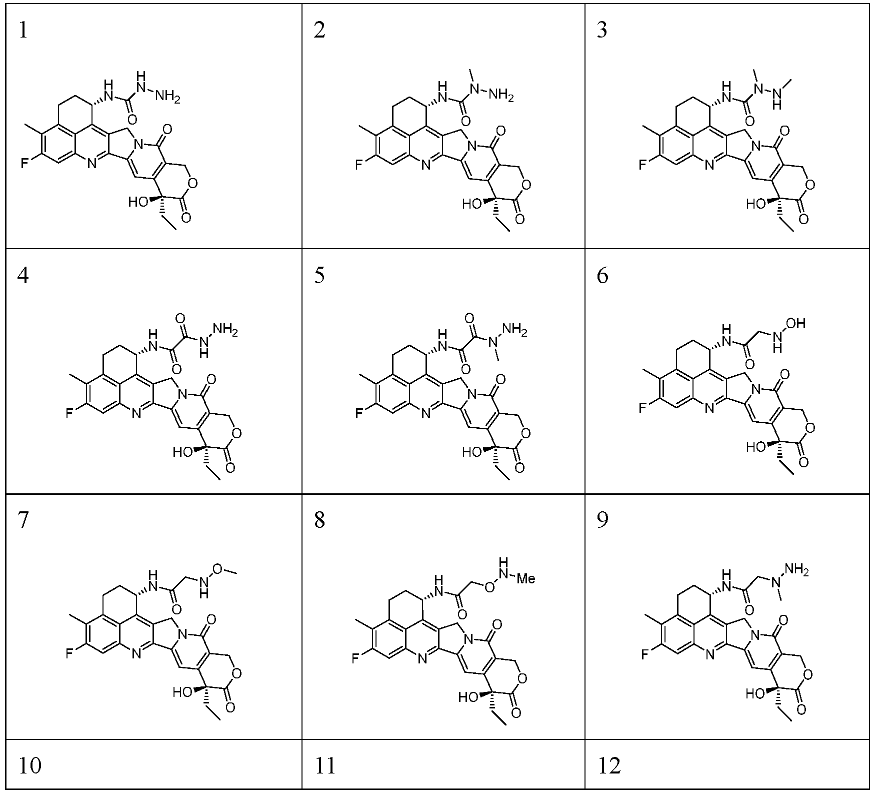

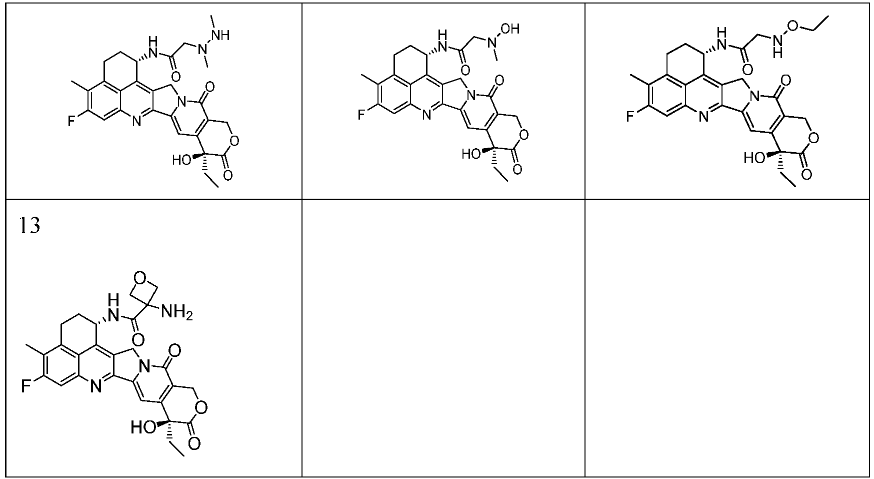

6. A method for preparing the compound according to any one of claims 1-5, selected from the following reaction schemes:
reaction scheme 1:
exatecan reacts with N,N'-carbonyldiimidazole, and then reacts with hydrazine or substituted hydrazine to afford a corresponding N-aminourea exatecan derivative;
reaction scheme 2:
substituted hydrazine reacts with oxalyl chloride, and then reacts with exatecan to afford a corresponding oxamide hydrazine exatecan derivative;
reaction scheme 3:
exatecan reacts with bromoacetic acid to afford bromoacetyl exatecan, and bromoacetyl exatecan condenses with an amine compound to afford a corresponding amide exatecan derivative;
reaction scheme 4:
exatecan reacts directly with a carboxylic acid compound to afford a corresponding amide exatecan derivative.

7. A drug-linker compound represented by formula (II) or a pharmaceutically acceptable salt, a stereoisomer, or a prodrug thereof: 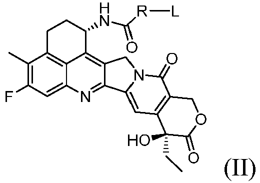
wherein, R is defined the same as the compound represented by formula (I);
L is -L₁-Q-L₂; R is connected to L₁ moiety;
wherein, L₁ is selected from 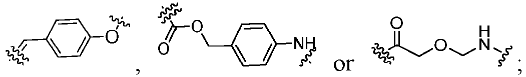 wherein, the position shown as " 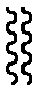 " indicates attachment to the R group, and the position shown as " 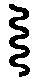 " indicates attachment to the Q group;
L₂ is selected from 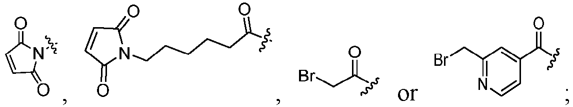
Q is selected from Val-Cit, Val-Ala, Ala-Ala-Asn, Gly-Gly-Phe-Gly, Gly-Lys, Gly-Gly-lys, (CH₂)ₘ₁O(CH₂)ₘ₂ or (CH₂)ₘ₃, wherein, m1 and m2 are each independently selected from an integer of 1-4; preferably, m1 is 2; preferably, m2 is 2; preferably, m3 is 6; preferably, Q is selected from Val-Cit, Gly-Gly-Phe-Gly, (CH₂)₂O(CH₂)₂ or (CH₂)₆;
preferably, L₁ is 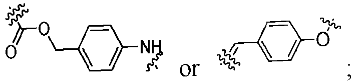
preferably, L₂ is

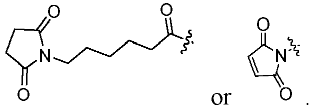

8. An antibody-drug conjugate compound represented by formula (III) or a pharmaceutically acceptable salt thereof: 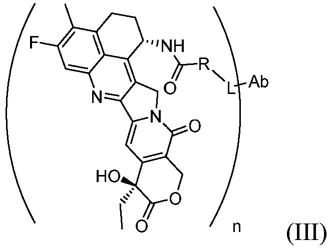
wherein, R is defined the same as formula (I), L is defined the same as formula (II);
Ab is an antibody for a tumor associated antigen, n is selected from an integer of 1-8;
preferably, the tumor associated antigen is selected from Her2, Trop2, 5T4, ROR1 or B7-H3;
preferably, the antibody-drug conjugate compound represented by formula (III) is selected from the following compounds: 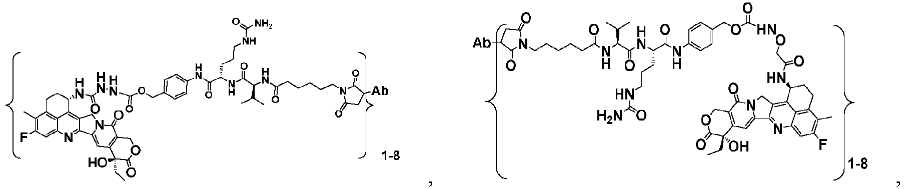 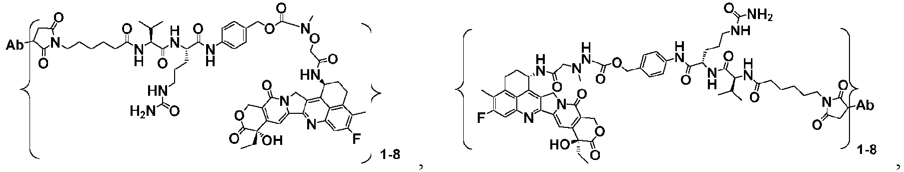 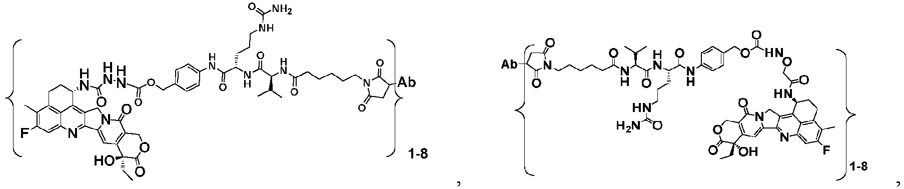 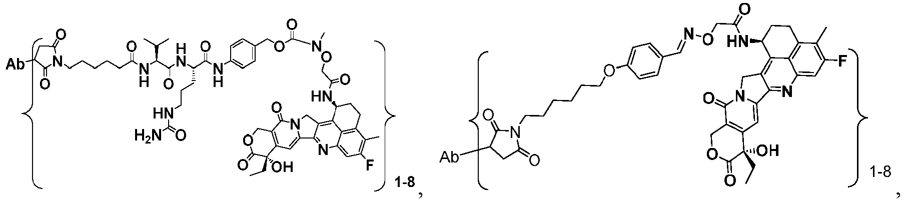 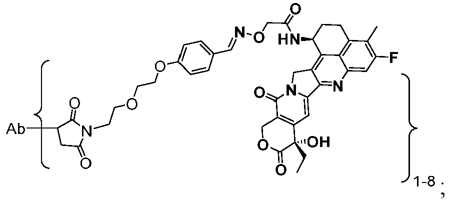
preferably, the antibody-drug conjugate compound represented by formula (III) is selected from the following compounds:

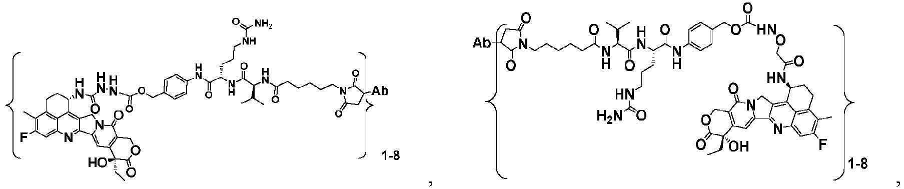

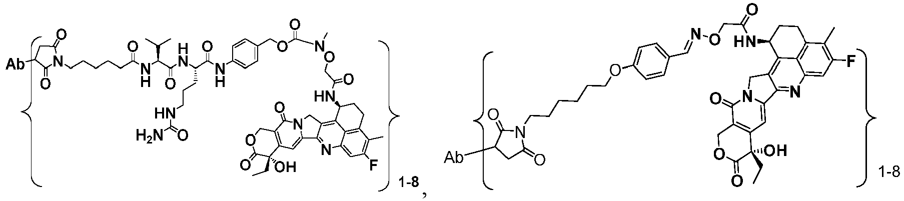

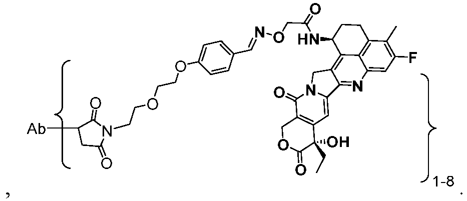

9. A pharmaceutical composition, **characterized in that**, it comprises the compound according to any one of claims 1-5, the drug-linker compound according to claim 7 or pharmaceutically acceptable salt, stereoisomer, prodrug thereof or the antibody-drug conjugate according to claim 8, and a pharmaceutically acceptable excipient.

10. Use of the compound according to any one of claims 1-5, the drug-linker compound according to claim 7 or pharmaceutically acceptable salt, stereoisomer, prodrug thereof, or the antibody-drug conjugate according to claim 8 or the pharmaceutical composition according to claim 9 in the manufacture of a medicament for treating a cancer;
preferably, the cancer includes gastric cancer, esophageal cancer, breast cancer and lung adenocarcinoma.

11. A method for treating a cancer comprising the step of administering to a patient in need thereof the compound according to any one of claim 1-5, the drug-linker compound according to claim 7 or pharmaceutically acceptable salt, stereoisomer, prodrug thereof or the antibody-drug conjugate according to claim 8 or the pharmaceutical composition according to claim 9.
